# EUROPÄISCHE PATENTSCHRIFT

(11) **EP 1 716 099 B1**
(45) Veröffentlichungstag und Bekanntmachung des Hinweises auf die Patenterteilung: **05.03.2014**
(21) Anmeldenummer: 05701115.7
(22) Anmeldetag: 22.01.2005
(51) Int. Cl.: C07C 233/66, C07D 231/14, C07D 231/16, C07D 327/06, A01N 43/56, A01N 37/18

(54) **HALOALKYLCARBOXAMIDE ZUR BEKÄMPFUNG VON MIKROORGANISMEN**
HALOALKYLCARBOXAMIDES FOR COMBATTING MICROORGANISMS
HALOALKYLCARBOXAMIDES POUR COMBATTRE LES MICROORGANISMES

(30) Priorität: 06.02.2004 DE 102004005786
(43) Veröffentlichungstag der Anmeldung: 02.11.2006
(73) Patentinhaber: Bayer CropScience Aktiengesellschaft, 40789 Monheim (DE)
(72) Erfinder: DUNKEL, Ralf, 69001 Lyon (FR); ELBE, Hans-Ludwig, 42329 Wuppertal (DE); HARTMANN, Benoit, 40764 Langenfeld (DE); GREUL, Jörg, Nico, 42799 Leichlingen (DE); ILG, Kerstin, 51061 Köln (DE); WACHENDORFF-NEUMANN, Ulrike, 56566 Neuwied (DE); DAHMEN, Peter, 41470 Neuss (DE); KUCK, Karl-Heinz, 40764 Langenfeld (DE)
(86) Internationale Anmeldenummer: PCT/EP2005/000608
(87) Internationale Veröffentlichungsnummer: WO 2005/075411

(56) Entgegenhaltungen:
- EP-A- 0 545 099
- EP-A- 0 589 301
- WO-A-02/38542
- COREY, ELIAS J. ET AL: "Asymmetric synthesis of .alpha.-amino acids. II. New systems for highly specific asymmetric synthesis with conservation of the chiral reagent" JOURNAL OF THE AMERICAN CHEMICAL SOCIETY , 92(8), 2488-501 CODEN: JACSAT; ISSN: 0002-7863, 1970, XP002323231

## Beschreibung

Die vorliegende Erfindung betrifft neue Haloalkylcarboxamide, mehrere Verfahren zu deren Herstellung und deren Verwendung zur Bekämpfung von unerwünschten Mikroorganismen.

Es ist bereits bekannt, dass zahlreiche Carboxamide fungizide Eigenschaften besitzen (vgl. z.B. WO 03/010149, WO 02/059086, EP-A 0 824 099, EP-A 0 737 682, EP-A 0 591 699, EP-A 0 589 301, EP-A 0 545 099, DE-A 24 09 011, DE-A 20 06 472, JP-A 2001-302605, JP-A 10-251240, JP-A 8-176112, JP-A 8-92223 und JP-A 53-72823). So sind bereits zahlreiche Alkylcarboxamide bekannt geworden, die im Alkylteil nicht substituiert sind, wie z.B. N-Allyl-N-[2-(1,3-dimethylbutyl)phenyl]-1-methyl-3-(trifluormethyl)-1H-pyrazol-4-carboxamid aus WO 02/059086, N-[2-(1,3-Dimethylbutyl)-phenyl]-2,4-dimethyl-1,3-thiazol-5-carboxamid aus EP-A 0 824 099 und 5-Fluor-1,3-dimethyl-N-[2-(1,3,3-trimethylbutyl)phenyl]-1H-pyrazol-4-carboxamid aus WO 03/010149. Die Wirksamkeit dieser Stoffe ist gut, lässt aber in manchen Fällen, z.B. bei niedrigen Aufwandmengen zu wünschen übrig.

Es wurden nun neue Haloalkylcarboxamide der Formel (I) 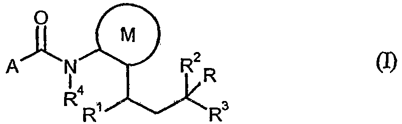 in welcher
- R: für Wasserstoff oder Halogen steht,
- R¹: für Wasserstoff oder Methyl steht,
- R²: für Methyl, Ethyl oder C₁-C₄-Halogenalkyl mit 1 bis 9 Fluor-, Chlor- und/oder Bromatomen steht,
- R³: für Halogen oder C₁-C₄-Halogenalkyl mit 1 bis 9 Fluor-, Chlor- und/oder Bromatomen steht,
- R⁴: für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkyl-sulfinyl, C₁-C₄-Halogenalkylsulfonyl, Halogen-C₁-C₄-alkoxy-C₁-C₄-alkyl, C₃-C₈-Halogen-cycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halo-gen-C₁-C₄-alkoxy-C₁-C₄-alkyl)carbonyl, (C₃-C₈-Halogencycloalkyl)carbonyl mit jeweils 1 bis 9 Fluor-, Chlor- und/oder Bromatomen; oder -C(=O)C(=O)R⁵, -CONR⁶R⁷ oder -CH₂NR⁸R⁹ steht,
- R⁵: für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halo-gencycloalkyl mit jeweils 1 bis 9 Fluor-, Chlor- und/oder Bromatomen steht,
- R⁶ und R⁷: unabhängig voneinander jeweils für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogen-cycloalkyl mit jeweils 1 bis 9 Fluor-, Chlor- und/oder Bromatomen stehen,
- R⁶ und R⁷: außerdem gemeinsam mit dem Stickstoffatom, an das sie gebunden sind, einen gegebenen-falls einfach oder mehrfach, gleich oder verschieden durch Halogen oder C₁-C₄-Alkyl substituierten gesättigten Heterocyclus mit 5 bis 8 Ringatomen bilden, wobei der Hetero-cyclus 1 oder 2 weitere, nicht benachbarte Heteroatome aus der Reihe Sauerstoff, Schwefel oder NR¹⁰ enthalten kann,
- R⁸ und R⁹: unabhängig voneinander für Wasserstoff, C₁-C₈-Alkyl, C₃-C₈-Cycloalkyl; C₁-C₈-Halogen-alkyl, C₃-C₈-Halogencycloalkyl mit jeweils 1 bis 9 Fluor-, Chlor- und/oder Bromatomen stehen,
- R⁸ und R⁹: außerdem gemeinsam mit dem Stickstoffatom, an das sie gebunden sind, einen gegebenen-falls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹⁰ enthalten kann,
- R¹⁰: für Wasserstoff oder C₁-C₆-Alkyl steht,
- M: für einen jeweils einfach durch R¹¹ substituierten Phenyl-,
- R¹¹: für Wasserstoff, Fluor, Chlor, Methyl, iso-Propyl, Methylthio oder Trifluormethyl steht,
- A: für den Rest der Formel (A1) 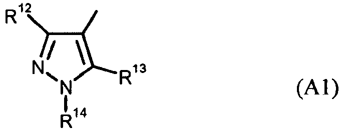 steht, in welcher
- R¹²: für Wasserstoff, Cyano, Halogen, Nitro, C₁-C₄-Alkyl, C₁-C₄-Alkoxy, C₁-C₄-Alkyl-thio, C₃-C₆-Cycloalkyl, C₁-C₄-Halogenalkyl, C₁-C₄-Halogenalkoxy oder C₁-C₄-Halo-genalkylthio mit jeweils 1 bis 5 Halogenatomen, Aminocarbonyl oder Aminocarbonyl-C₁-C₄-alkyl steht,
- R¹³: für Wasserstoff, Halogen, Cyano, C₁-C₄-Alkyl, C₁-C₄-Alkoxy oder C₁-C₄-Alkylthio steht,
- R¹⁴: für Wasserstoff, C₁-C₄-Alkyl, Hydroxy-C₁-C₄-alkyl, C₂-C₆-Alkenyl, C₃-C₆-Cyclo-alkyl, C₁-C₄-Alkylthio-C₁-C₄-alkyl, C₁-C₄-Alkoxy-C₁-C₄-alkyl, C₁-C₄-Halogenalkyl), C₁-C₄-Halogenalkylthio-C₁-C₄-alkyl, C₁-C₄-Halogenalkoxy-C₁-C₄-alkyl mit jeweils 1 bis 5 Halogenatomen, oder Phenyl steht,

Weiterhin wurde gefunden, dass man Haloalkylcarboxamide der Formel (I) erhält, indem man Weiterhin wurde gefunden, dass man Hexylcarboxanilide der Formel (I) erhält, indem man
a) Carbonsäure-Derivate der Formel (II) 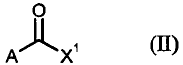 in welcher
   - A: die oben angegebenen Bedeutungen hat und
   - X¹: für Halogen oder Hydroxy steht,
   mit Anilin-Derivaten der Formel (III) 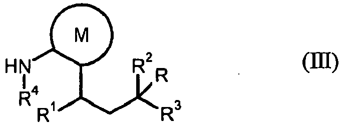 in welcher R, R¹, R², R³, R⁴ und M die oben angegebenen Bedeutungen haben, gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt,
   oder
b) Hexylcarboxanilide der Formel (I-a) 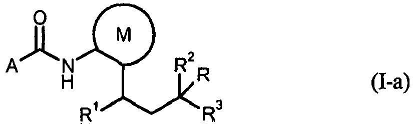 in welcher R, R¹, R², R³, M und A die oben angegebenen Bedeutungen haben mit Halogeniden der Formel (IV) 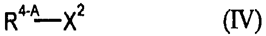 in welcher
   - X²: für Chlor, Brom oder Iod steht,
   - R^{4-A}: für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halo-genalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)car-bonyl-C₁-C₃-alkyl; Halogen-(C₁-C₃-alkyl)carbonyl-C₁-C₃-alkyl, Halogen-(C₁-C₃-alk-oxy)carbonyl-C₁-C₃-alkyl mit jeweils 1 bis 13 Fluor-, Chlor- und/oder Bromatomen; (C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₈-Halogencycloalkyl)carbonyl mit jeweils 1 bis 9 Fluor-, Chlor- und/oder Bromatomen; oder -C(=O)C(=O)R⁵, -CONR⁶R⁷ oder -CH₂NR⁸R⁹ steht,
   wobei R⁵, R⁶, R⁷, R⁸ und R⁹ die oben angegebenen Bedeutungen haben,
   in Gegenwart einer Base und in Gegenwart eines Verdünnungsmittels umsetzt.

Schließlich wurde gefunden, dass die neuen Haloalkylcarboxamide der Formel (I) sehr gute mikrobizide Eigenschaften besitzen und zur Bekämpfung unerwünschter Mikroorganismen sowohl im Pflanzenschutz als auch im Materialschutz verwendbar sind.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Haloalkylcarboxamide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- R: steht bevorzugt für Wasserstoff, Fluor, Chlor oder Brom.
- R: steht besonders bevorzugt für Wasserstoff.
- R: steht außerdem besonders bevorzugt für Fluor oder Chlor.

- R¹: steht bevorzugt für Wasserstoff.
- R¹: steht außerdem bevorzugt für Methyl.
- R²: steht bevorzugt für Methyl, Ethyl oder für jeweils einfach oder mehrfach, gleich oder verschieden durch Fluor, Chlor oder Brom substituiertes Methyl, Ethyl, n- oder iso-Propyl, n-, iso-, sec- oder tert-Butyl.
- R²: steht besonders bevorzugt für Methyl, Ethyl, Trifluormethyl, Difluormethyl, Fluormethyl, Trichlormethyl, Dichlormethyl, Chlormethyl, Chlorfluormethyl, Fluordichlormethyl, Difluorchlormethyl, Pentafluorethyl, 1-Fluorethyl, 2-Fluorethyl, 2,2-Difluorethyl, 2,2,2-Trifluorethyl, 2-Chlor-2-fluorethyl, 2-Chlor-2,2difluorethyl, 2-Chlor-2,2-difluorethyl, 2-Dichlor-2-fluorethyl, 2,2,2-Trichlorethyl, 1-Chlorbutyl, Heptafluor-n-propyl oder Heptafluorisopropyl.
- R²: steht ganz besonders bevorzugt für Methyl, Ethyl oder Trifluormethyl.
- R²: steht insbesondere bevorzugt für Methyl.
- R²: steht außerdem insbesondere bevorzugt für Ethyl.
- R²: steht außerdem insbesondere bevorzugt für Trifluormethyl.

- R³: steht bevorzugt für Fluor, Chlor, Brom, Iod oder für jeweils einfach oder mehrfach, gleich oder verschieden durch Fluor, Chlor oder Brom substituiertes Methyl, Ethyl, n- oder isoPropyl, n-, iso-, sec- oder tert-Butyl.
- R³: steht besonders bevorzugt für Fluor, Chlor, Brom, Trifluormethyl, Difluormethyl, Fluormethyl, Trichlormethyl, Dichlormethyl, Chlormethyl, Chlorfluormethyl, Fluordichlormethyl, Difluorchlormethyl, Pentafluorethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2-Chlor-2,2-difluorethyl, 2-Dichlor-2-fluorethyl, 2,2,2-Trichlorethyl, 1-Chlorbutyl, Heptafluor-n-propyl oder Heptafluorisopropyl.
- R³: steht ganz besonders bevorzugt für Chlor oder Trifluormethyl.
- R³: steht insbesondere bevorzugt für Chlor.
- R³: steht außerdem insbesondere bevorzugt für Trifluormethyl.

- R⁴: steht bevorzugt für Wasserstoff, C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-al-kyl mit jeweils 1 bis 13 Fluor-, Chlor- und/oder Bromatomen; (C₁-C₆-Alkyl)carbonyl, (C₁-C₄-Alkoxy)carbonyl, (C₁-C₃-Alkoxy-C₁-C₃-alkyl)carbonyl, (C₃-C₆-Cycloalkyl)carbonyl; (C₁-C₄-Halogenalkyl)carbonyl, (C₁-C₄-Halogenakoxy)carbonyl, (Halogen-C₁-C₃-akoxy-C₁-C₃-alkyl)carbonyl, (C₃-C₆-Halogencycloalkyl)carbonyl mit jeweils 1 bis 9 Fluor-, Chlor- und/oder Bromatomen; oder -C(=O)C(=O)R⁵, -CONR⁶R⁷ oder -CH₂NR⁸R⁹.
- R⁴: steht besonders bevorzugt für Wasserstoff, Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Difluorchlormethylthio, Trifluormethylthio, Trifluormethylsulfinyl, Trifluormethylsulfonyl, Trifluormethoxymethyl; Formyl, -CH₂-CHO, -(CH₂)₂-CHO, -CH₂-CO-CH₃, -CH₂-CO-CH₂CH₃, -CH₂-CO-CH(CH₃)₂, -(CH₂)₂-CO-CH₃, -(CH₂)₂-CO-CH₂CH₃, -(CH₂)₂-CO-CH(CH₃)₂, -CH₂-CO₂CH₃, -CH₂-CO₂CH₂CH₃, -CH₂-CO₂CH(CH₃)₂, -(CH₂)₂-CO₂CH₃, -(CH₂)₂-CO₂CH₂CH₃, -(CH₂)₂-CO₂CH(CH₃)₂, -CH₂CO-CF₃, -CH₂-CO-CCl₃, -CH₂-CO-CH₂CF₃, -CH₂-CO-CH₂CCl₃, -(CH₂)₂-CO-CH₂CF₃, -(CH₂)₂-CO-CH₂CCl₃, -CH₂-CO₂CH₂CF₃, -CH₂-CO₂CF₂CF₃, -CH₂-CO₂CH₂CCl₃, -CH₂-CO₂CCl₂CCl₃, -(CH₂)₂-CO₂CH₂CF₃, -(CH₂)₂-CO₂CF₂CF₃, -(CH₂)₂-CO₂CH₂CCl₃, -(CH₂)₂-CO₂CCl₂CCl₃; Methylcarbonyl, Ethylcarbonyl, n-Propylcarbonyl, iso-Propylcarbonyl, tert-Butylcarbonyl, Methoxycarbonyl, Ethoxycarbonyl, tert-Butoxycarbonyl, Cyclopropylcarbonyl; Trifluormethylcarbonyl, Trifluormethoxycarbonyl, oder -C(=O)C(=O)R⁵, -CONR⁶R⁷ oder -CH₂NR⁸R⁹.
- R⁴: steht ganz besonders bevorzugt für Wasserstoff, Methyl, Methoxymethyl, Formyl, -CH₂-CHO, -(CH₂)₂-CHO, -CH₂-CO-CH₃, -CH₂-CO-CH₂CH₃, -CH₂-CO-CH(CH₃)₂, -C(=O)CHO, -C(=O)C(=O)CH₃, -C(=O)C(=O)CH₂OCH₃, -C(=O)CO₂CH₃, -C(=O)CO₂CH₂CH₃.
- R⁵: steht bevorzugt für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R⁵: steht besonders bevorzugt für Wasserstoff, Methyl, Ethyl, n- oder iso-Propyl, tert-Butyl, Methoxy, Ethoxy, n- oder iso-Propoxy, tert-Butoxy, Methoxymethyl, Cyclopropyl; Trifluormethyl, Trifluormethoxy.
- R⁶ und R⁷: stehen unabhängig voneinander bevorzugt für Wasserstoff, C₁-C₆-Alkyl, C₁-C₃-Alkoxy-C₁-C₃-alkyl, C₃-C₆-Cycloalkyl; C₁-C₄-Halogenalkyl, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R⁶ und R⁷: bilden außerdem gemeinsam mit dem Stickstoffatom, an das sie gebunden sind, bevorzugt einen gegebenenfalls einfach bis vierfach, gleich oder verschieden durch Halogen oder C₁-C₄-Alkyl substituierten gesättigten Heterocyclus mit 5 oder 6 Ringatomen, wobei der Heterocyclus 1 oder 2 weitere, nicht benachbarte Heteroatome aus der Reihe Sauerstoff, Schwefel oder NR¹⁰ enthalten kann.
- R⁶ und R⁷: stehen unabhängig voneinander besonders bevorzugt für Wasserstoff, Methyl, Ethyl, n-oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R⁶ und R⁷: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R¹⁰ substituiert sein kann.
- R⁸ und R⁹: stehen unabhängig voneinander bevorzugt für Wasserstoff, C₁-C₆-Alkyl, C₃-C₆-Cycloalkyl; C₁-C₄-Halogenalkyl, C₃-C₆-Halogencycloalkyl mit jeweils 1 bis 9 Fluor-, Chlor- und/oder Bromatomen.
- R⁸ und R⁹: bilden außerdem gemeinsam mit dem Stickstoffatom, an das sie gebunden sind, bevorzugt einen gegebenenfalls einfach oder mehrfach, gleich oder verschieden durch Halogen oder C₁-C₄-Alkyl substituierten gesättigten Heterocyclus mit 5 oder 6 Ringatomen, wobei der Heterocyclus 1 oder 2 weitere, nicht benachbarte Heteroatome aus der Reihe Sauerstoff, Schwefel oder NR¹⁰ enthalten kann.
- R⁸ und R⁹: stehen unabhängig voneinander besonders bevorzugt für Wasserstoff, Methyl, Ethyl, n-oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R⁸ und R⁹: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R¹⁰ substituiert sein kann.
- R¹⁰: steht bevorzugt für Wasserstoff oder C₁-C₄-Alkyl.
- R¹⁰: steht besonders bevorzugt für Wasserstoff, Methyl, Ethyl, n- oder iso-Propyl, n-, iso-, sec-oder tert-Butyl.
- M: steht bevorzugt für einen der folgenden Cyclen 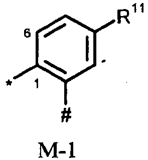
wobei die mit "*" markierte Bindung mit dem Amid, die mit "#" markierte Bindung mit dem Haloalkylrest verknüpft ist.
- R¹¹: steht bevorzugt für Wasserstoff, Fluor, Chlor, Methyl oder Trifluormethyl.
- R¹¹: steht besonders bevorzugt für Wasserstoff oder Chlor.
- R¹¹: steht für den Fall, dass M für M-2, M-3, M-4 oder M-5 steht, außerdem bevorzugt für Fluor, wobei Fluor besonders bevorzugt in 6-Position (M-2, M-3) oder in 3-Position (M-4, M-5) steht.
- R¹¹: steht für den Fall, dass M für M-2, M-3, M-4 oder M-5 steht, außerdem bevorzugt für Chlor, wobei Chlor besonders bevorzugt in 6-Position (M-2, M-3) oder in 3-Position (M-4, M-5) steht.
- R¹¹: steht für den Fall, dass M für M-2, M-3, M-4 oder M-5 steht, außerdem bevorzugt für Methyl, wobei Methyl besonders bevorzugt in 4-Position (M-2) oder in 3-Position (M-3, M-4, M-5) steht.
- R¹¹: steht für den Fall, dass M für M-6 steht, außerdem bevorzugt für Methyl, wobei Methyl besonders bevorzugt in 3-Position steht.
- R¹¹: steht für den Fall, dass M für M-6 steht, außerdem bevorzugt für Trifluormethyl, wobei Tri-fluormethyl besonders bevorzugt in 3-Position steht.
- R¹¹: steht für den Fall, dass M für M-9 steht, außerdem bevorzugt für Methyl, wobei Methyl besonders bevorzugt in 4-Position steht.
- R¹¹: steht für den Fall, dass M für M-9 steht, außerdem bevorzugt für Trifluormethyl, wobei Trifluormethyl besonders bevorzugt in 4-Position steht.
- R¹¹: steht für den Fall, dass M für M-10 steht, außerdem bevorzugt für Methyl, wobei Methyl besonders bevorzugt in 3-Position steht.
- R¹¹: steht für den Fall, dass M für M-10 steht, außerdem bevorzugt für Trifluormethyl, wobei Tri-fluormethyl besonders bevorzugt in 3-Position steht.
- R¹¹: steht für den Fall, dass M für M-11 steht, außerdem bevorzugt für Methyl, wobei Methyl besonders bevorzugt in 3-Position steht.
- R¹¹: steht für den Fall, dass M für M-11 steht, außerdem bevorzugt für Trifluormethyl, wobei Trifluormethyl besonders bevorzugt in 3-Position steht.

- R¹²: steht bevorzugt für Wasserstoff, Cyano, Fluor, Chlor, Brom, Iod, Methyl, Ethyl, iso-Propyl, Methoxy, Ethoxy, Methylthio, Ethylthio, Cyclopropyl, C₁-C₂-Halogenalkyl, C₁-C₂-Halo-genalkoxy mit jeweils 1 bis 5 Fluor, Chlor und/oder Bromatomen, Trifluormethylthio, Difluormethylthio, Aminocarbonyl, Aminocarbonylmethyl oder Aminocarbonylethyl.
- R¹²: steht besonders bevorzugt für Wasserstoff, Fluor, Chlor, Brom, Iod, Methyl, Ethyl, isoPropyl, Monofluormethyl, Monofluorethyl, Difluormethyl, Trifluormethyl, Difluorchlormethyl, Trichlormethyl, Dichlormethyl, Cyclopropyl, Methoxy, Ethoxy, Trifluormethoxy, Trichlormethoxy, Methylthio, Ethylthio, Trifluormethylthio oder Difluormethylthio.
- R¹²: steht ganz besonders bevorzugt Wasserstoff, Fluor, Chlor, Brom, Iod, Methyl, iso-Propyl, Monofluormethyl, Monofluorethyl, Difluormethyl, Trifluormethyl, Difluorchlormethyl oder Trichlormethyl.
- R¹²: steht insbesondere bevorzugt für Methyl, Difluormethyl, Trifluormethyl oder 1-Fluorethyl.
- R¹³: steht bevorzugt für Wasserstoff, Fluor, Chlor, Brom, Iod, Methyl, Ethyl, Methoxy, Ethoxy, Methylthio oder Ethylthio.
- R¹³: steht besonders bevorzugt für Wasserstoff, Fluor, Chlor, Brom, Iod oder Methyl.
- R¹³: steht ganz besonders bevorzugt für Wasserstoff, Fluor, Chlor oder Methyl.
- R¹⁴: steht bevorzugt für Wasserstoff, Methyl, Ethyl, n-Propyl, iso-Propyl, C₁-C₂-Halogenalkyl mit 1 bis 5 Fluor, Chlor und/oder Bromatomen, Hydroxymethyl, Hydroxyethyl, Cyclopropyl, Cyclopentyl, Cyclohexyl oder Phenyl.
- R¹⁴: steht besonders bevorzugt für Wasserstoff, Methyl, Ethyl, iso-Propyl, Trifluormethyl, Difluormethyl, Hydroxymethyl, Hydroxyethyl oder Phenyl.
- R¹⁴: steht ganz besonders bevorzugt für Wasserstoff, Methyl, Trifluormethyl oder Phenyl.
- R¹⁴: steht insbesondere bevorzugt für Methyl.

Bevorzugt sind solche Verbindungen der Formel (I), in welcher alle Reste jeweils die oben genannten bevorzugten Bedeutungen haben.

Besonders bevorzugt sind solche Verbindungen der Formel (I), in welcher alle Reste jeweils die oben genannten besonders bevorzugten Bedeutungen haben.

Bevorzugt und jeweils als Teilmenge der oben genannten Verbindungen der Formel (I) zu verstehen sind folgende Gruppen von neuen Carboxamiden:
Gruppe 1: Haloalkylcarboxamide der Formel (I-a) 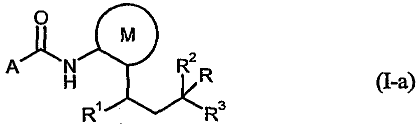 in welcher R, R¹, R², R³, M und A die oben angegebenen Bedeutungen haben.
Gruppe 2: Haloalkylcarboxamide der Formel (I-b) 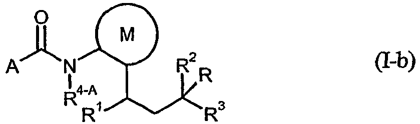 in welcher R, R¹, R², R³, R^{4-A}, M und A die oben angegebenen Bedeutungen haben.
   - R^{4-A}: steht bevorzugt für C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₈-Halogen-cycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen;
   (C₁-C₆-Alkyl)carbonyl, (C₁-C₄-Alkoxy)carbonyl, (C₁-C₃-Alkoxy-C₁-C₃-alkyl)carbonyl, (C₃-C₆-Cycloalkyl)carbonyl; (C₁-C₄-Halogenalkyl)carbonyl, (C₁-C₄-Halogenalkoxy)carbonyl, (Halogen-C₁-C₃-alkoxy-C₁-C₃-alkyl)carbonyl, (C₃-C₆-Halogencycloalkyl)carbonyl mit jeweils 1 bis 9 Fluor-, Chlor- und/oder Bromatomen; oder -C(=O)C(=O)R⁵, -CONR⁶R⁷ oder -CH²NR⁸R⁹.
   - R^{4-A}: steht besonders bevorzugt für Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, secoder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Difluorchlormethylthio, Trifluormethylthio, Trifluormethylsulfinyl, Trifluormethylsulfonyl, Trifluormethoxymethyl; Formyl, -CH₂-CHO, -(CH₂)₂-CHO, -CH₂-CO-CH₃, -CH₂-CO-CH₂CH₃, -CH₂-CO-CH(CH₃)₂, -(CH₂)₂-CO-CH₃, -(CH₂)₂-CO-CH₂CH₃, -(CH₂)₂-CO-CH(CH₃)₂, -CH₂-CO₂CH₃, -CH₂-CO₂CH₂CH₃, -CH₂-CO₂CH(CH₃)₂, -(CH₂)₂-CO₂CH₃, -(CH₂)₂-CO₂CH₂CH₃, -(CH₂)₂-CO₂CH(CH₃)₂, -CH₂CO-CF₃, -CH₂-CO-CCl₃, -CH₂-CO-CH₂CF₃, -CH₂CO-CH₂CCl₃, -(CH₂)₂-CO-CH₂CF₃, -(CH₂)₂-CO-CH₂CCl₃, -CH₂-CO₂CH₂CF₃, -CH₂-CO₂CF₂CF₃, -CH₂-CO₂CH₂CCl₃, -CH₂-CO₂CCl₂CCl₃, -(CH₂)₂-CO₂CH₂CF₃, -(CH₂)₂-CO₂CF₂CF₃, -(CH₂)₂-CO₂CH₂CCl₃, -(CH₂)₂-CO₂CCl₂CCl₃; Methylcarbonyl, Ethylcarbonyl, n-Propylcarbonyl, iso-Propylcarbonyl, tert-Butylcarbonyl, Methoxycarbonyl, Ethoxycarbonyl, tert-Butoxycarbonyl, Cyclopropylcarbonyl; Trifluormethylcarbonyl, Trifluormethoxycarbonyl, oder -C(=O)C(=O)R⁵, -CONR⁶R⁷ oder -CH₂NR⁸R⁹.
   - R^{4-A}: steht ganz besonders bevorzugt für Methyl, Methoxymethyl, Formyl, -CH₂-CHO, -(CH₂)₂-CHO, -CH₂-CO-CH₃, -CH₂-CO-CH₂CH₃, -CH₂-CO-CH(CH₃)₂, -C(=O)CHO, -C(=O)C(=O)CH₃, -C(=O)C(=O)CH₂OCH₃, -C(=O)CO₂CH₃, -C(=O)CO₂CH₂CH₃.
Gruppe 3: Haloalkylcarboxamide der Formel (I-c) 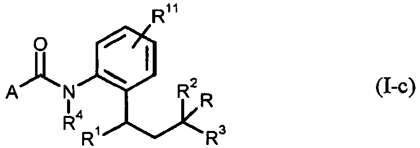 in welcher R, R¹, R², R³, R⁴, R¹¹ und A die oben angegebenen Bedeutungen haben. Bevorzugt sind Haloalkylcarboxamide der Formel (I-c), in welcher R⁴ für Wasserstoff steht. Bevorzugt sind Haloalkylcarboxamide der Formel (I-c), in welcher R¹¹ für Wasserstoff steht. Bevorzugt sind Haloalkylcarboxamide der Formel (I-c), in welcher R⁴ und R¹¹ jeweils für Wasserstoff stehen.

Hervorgehoben sind Verbindungen der Formel (I) (und ebenso der Gruppen 1 bis 3), in welcher R⁴ für Wasserstoff steht.

Hervorgehoben sind Verbindungen der Formel (I) (und ebenso der Gruppen 1 bis 3), in welcher R⁴ für Formyl steht.

Hervorgehoben sind außerdem Verbindungen der Formel (I) (und ebenso der Gruppen 1 bis 3), in welcher R⁴ für -C(=O)C(=O)R⁵ steht, wobei R⁵ die oben angegebenen Bedeutungen hat.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können. So schließt die Definition Dialkylamino auch eine unsymmetrisch durch Alkyl substituierte Aminogruppe wie z.B. Methyl-ethylamino ein.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können zwischen den jeweiligen Bereichen und Vorzugsbereichen beliebig kombiniert werden. Sie gelten für die Endprodukte sowie für die Vor- und Zwischenprodukte entsprechend. Insbesondere können die in den Gruppen 1 bis 6 genannten Verbindungen sowohl mit den allgemeinen wie auch mit bevorzugten, besonders bevorzugten usw. Bedeutungen kombiniert werden, wobei auch hier jeweils alle Kombinationen zwischen den Vorzugsbereichen möglich sind.

### Beschreibung der erfindungsgemäßen Verfahren zum Herstellen der Hexylcarboxanilide der Formel (I) sowie der Zwischenprodukte

### Verfahren (a)

Die zur Durchführung des erfindungsgemäßen Verfahrens (a) als Ausgangsstoffe benötigten Carbonsäure-Derivate sind durch die Formel (II) allgemein definiert. In dieser Formel (II) hat A bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für A angegeben wurden. X¹ steht bevorzugt für Chlor, Brom oder Hydroxy.

Die Carbonsäure-Derivate der Formel (II) sind größtenteils bekannt und/oder lassen sich nach bekannten Verfahren herstellen (vgl. WO 93/11117, EP-A 0 545 099, EP-A 0 589 301 und EP-A 0 589 313).

Die zur Durchführung des erfindungsgemäßen Verfahrens (a) als Ausgangsstoffe weiterhin benötigten Anilin-Derivate sind durch die Formel (III) allgemein definiert. In dieser Formel (III) haben R, R¹, R¹, R³, R⁴ und M bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) für diese Reste als bevorzugt, besonders bevorzugt bzw. ganz besonders bevorzugt angegeben wurden.

### Anilin-Derivate der Formel (III-a)

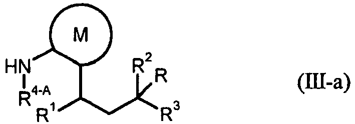

in welcher R, R¹, R², R³, R^{4-A} und M die oben angegebenen Bedeutungen haben,
werden erhalten, indem man

### c) Anilin-Derivate der Formel (III-b)

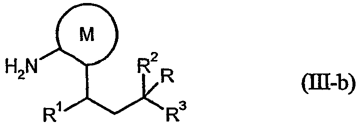

in welcher R, R¹, R², R³ und M die oben angegebenen Bedeutungen haben,
mit Halogeniden der Formel (IV) 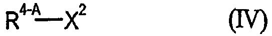 in welcher R^{4-A} und X² die oben angegebenen Bedeutungen haben,
in Gegenwart einer Base und in Gegenwart eines Verdünnungsmittels umsetzt.

### Anilin-Derivate der Formel (III-c)

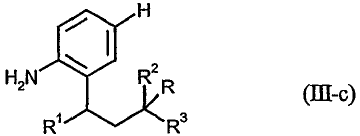

in welcher R, R¹, R² und R³ die oben angegebenen Bedeutungen haben, werden erhalten, indem man

### d) Anilin-Derivate der Formel (III-d)

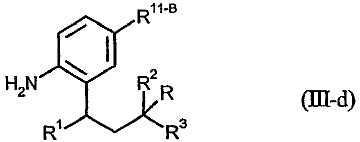

in welcher
R, R¹, R² und R³ die oben angegebenen Bedeutungen haben,
R^{11-B} für Fluor oder Chlor steht,
in Gegenwart eines Reduktionsmittels, eines Katalysators und eines Verdünnungsmittels umsetzt.

### Anilin-Derivate der Formel (III-e)

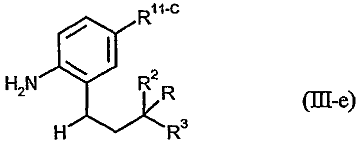

in welcher
R, R² und R³ die oben angegebenen Bedeutungen haben,
R^{11-C} für Fluor, Chlor, Methyl, iso-Propyl, Methylthio oder Trifluormethyl steht, werden erhalten, indem man

### e) Haloalkanonaniline der Formel (V)

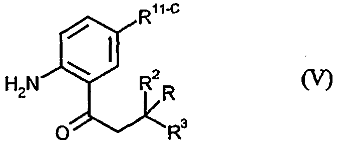

in welcher R, R², R³ und R^{11-C} die oben angegebenen Bedeutungen haben,
mit Hydrazin oder Hydrazinhydrat in Gegenwart einer Base (z.B. Alkali- oder Erdalkalimetallhydroxide wie Natriumhydroxid oder Kaliumhydroxid) und gegebenenfalls in Gegenwart eines Verdünnungsmittels umsetzt.

### Haloalkanonaniline der Formel (V)

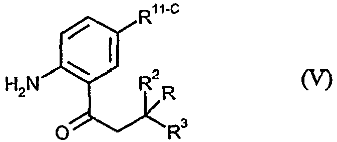

in welcher R, R², R³ und R^{11-C} die oben angegebenen Bedeutungen haben, werden erhalten, indem man

### f) geschützte Haloalkanonaniline der Formel (VI)

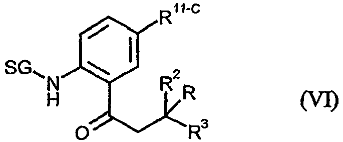

in welcher
R, R², R³ und R^{11-C} die oben angegebenen Bedeutungen haben,
- SG: für eine Schutzgruppe, bevorzugt Piv (tert-Butylcarbonyl), Boc (tert-Butoxycarbonyl-), Cbz (Benzyloxycarbonyl-), Trifluoracetyl-, Fmoc (9-Fluorenylmethoxycarbonyl-) oder Troc (2,2,2-Trichlorethoxycarbonyl-), steht,
in Gegenwart einer Säure (z.B. Salzsäure) und gegebenenfalls in Gegenwart eines Verdünnungsmittels umsetzt.

### Geschützte Haloalkanonaniline der Formel (VI)

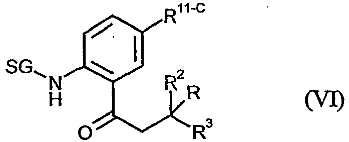

in welcher R, R², R³, R^{11-C} und SG die oben angegebenen Bedeutungen haben, werden erhalten, indem man

### g) Geschützte Aniline der Formel (VII)

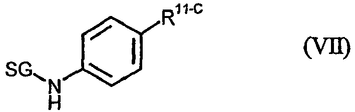

in welcher R^{11-C} und SG die oben angegebenen Bedeutungen haben,
mit einem Ester der Formel (VIII) 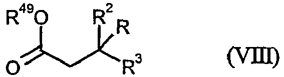 in welcher
R, R² und R³ die oben angegebenen Bedeutungen haben,
R⁴⁹ für C₁-C₄-Alkyl, bevorzugt Methyl oder Ethyl, steht,
in Gegenwart eines Verdünnungsmittels und metallorganischer Basen umsetzt.

Geschützte Aniline der Formel (VII) und Ester der Formel (VIII) sind bekannt.

### Anilin-Derivate der Formel (III-f)

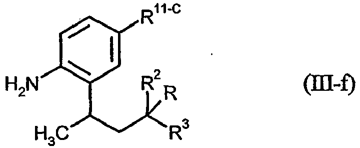

in welcher
R, R², R³ und R^{11-C} die oben angegebenen Bedeutungen haben, werden erhalten, indem man

### h) Alkene der Formel (IX)

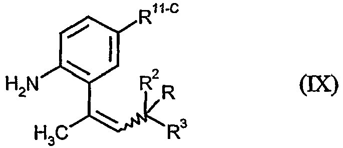

in welcher R, R², R³ und R^{11-C} die oben angegebenen Bedeutungen haben,
gegebenenfalls in Gegenwart eines Verdünnungsmittels und gegebenenfalls in Gegenwart eines Katalysators hydriert.

### Alkene der Formel (IX)

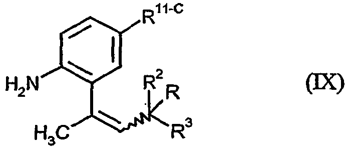

in welcher R, R², R³ und R^{11-C} die oben angegebenen Bedeutungen haben, werden erhalten, indem man

### i) Hydroxyalkylaniline der Formel (X)

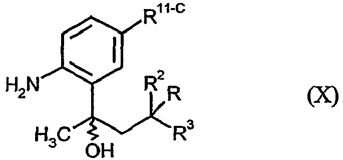

in welcher R, R², R³ und R^{11-C} die oben angegebenen Bedeutungen haben, gegebenenfalls in Gegenwart eines Verdünnungsmittels und gegebenenfalls in Gegenwart einer Säure dehydratisiert.

### Hydroxyalkylaniline der Formel (X)

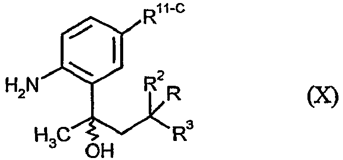

in welcher R, R², R³ und R^{11-C} die oben angegebenen Bedeutungen haben, werden erhalten, indem man

### k) Haloalkanonaniline der Formel (V)

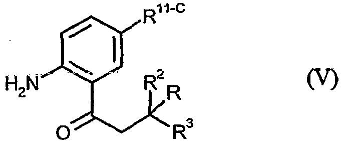

in welcher R, R², R³ und R^{11-C} die oben angegebenen Bedeutungen haben,
mit metallorganischen Verbindungen (z.B. Methylmagnesium Halogeniden) in Gegenwart eines Verdünnungsmittels umsetzt.

### Anilin-Derivate der Formel (III-g)

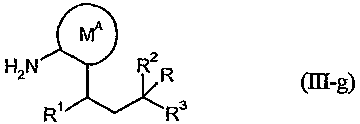

in welcher
R, R¹, R² und R³ die oben angegebenen Bedeutungen haben,
- M^{A}: für einen jeweils einfach durch R¹¹ substituierten Pyridin- oder Pyrimidin-Ring oder für einen einfach durch R^{11-A} substituierten Thiazol-Ring steht,
können analog oder nach bekannten Verfahren (vgl. EP-A 0 737 682) erhalten werden.

### Verfahren (b)

Verwendet man 3-(Difluormethyl)-1-methyl-N-[2-(4,4,4-trifluor-3-methylbutyl)phenyl]-1H-pyrazol-4-carboxamid und Ethyl-chlor(oxo)acetat als Ausgangsstoffe, so kann der Verlauf des erfindungsgemäßen Verfahrens (b) durch das folgende Formelschema veranschaulicht werden:

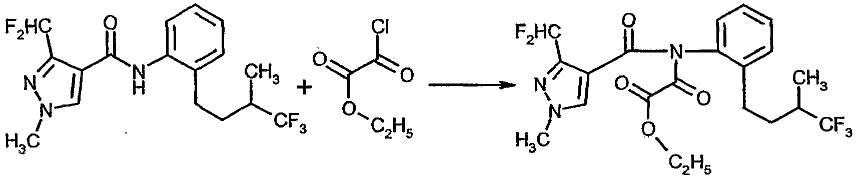

Die zur Durchführung des erfindungsgemäßen Verfahrens (b) als Ausgangsstoffe benötigten Hexylcarboxanilide sind durch die Formel (I-a) allgemein definiert. In dieser Formel (I-a) haben R, R¹, R², R³, M und A bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Hexylcarboxanilide der Formel (I-a) sind ebenfalls erfindungsgemäße Verbindungen und Gegenstand dieser Anmeldung. Sie können nach dem erfindungsgemäßen Verfahren (a) erhalten werden (mit R¹ = Wasserstoff).

Die zur Durchführung des erfindungsgemäßen Verfahrens (b) als Ausgangsstoffe weiterhin benötigten Halogenide sind durch die Formel (IV) allgemein definiert. In dieser Formel (IV) hat R^{4-A} bevorzugt, besonders bevorzugt bzw. ganz besonders bevorzugt diejenigen Bedeutungen, die bereits im Zusammenhang mit der Beschreibung der erfindungsgemäßen Verbindungen der Formel (I-b) als bevorzugt, besonders bevorzugt bzw. ganz besonders bevorzugt für diesen Rest angegeben wurden. X² steht bevorzugt für Chlor oder Brom.

Halogenide der Formel (IV) sind bekannt.

### Reaktionsbedingungen

Als Verdünnungsmittel zur Durchführung des erfindungsgemäßen Verfahrens (a) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (a) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (a) wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ), Triphenylphosphin/Tetrachlorkohlenstoff oder Brom-tripyrrolidinophosphonium-hexafluorophosphat.

Das erfindungsgemäße Verfahren (a) wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung des erfindungsgemäßen Verfahrens (a) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens (a) zur Herstellung der Verbindungen der Formel (I) setzt man pro mol des Carbonsäure-Derivates der Formel (II) im Allgemeinen 0,2 bis 5 mol, vorzugsweise 0,5 bis 2 mol an Anilin-Derivat der Formel (III) ein.

Als Verdünnungsmittel zur Durchführung der erfindungsgemäßen Verfahren (b) und (c) kommen alle inerten organischen Lösungsmittel in Betracht Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Die erfindungsgemäßen Verfahren (b) und (c) werden in Gegenwart einer Base durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (b) und (c) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens (b) zur Herstellung der Verbindungen der Formel (I) setzt man pro Mol des Hexylcarboxanilids der Formel (I-a) im Allgemeinen 0,2 bis 5 Mol, vorzugsweise 0,5 bis 2 Mol an Halogenid der Formel (IV) ein.

Zur Durchführung des erfindungsgemäßen Verfahrens (c) zur Herstellung der Verbindungen der Formel (III-a) setzt man pro Mol des Anilin-Derivates der Formel (III-b) im Allgemeinen 0,2 bis 5 Mol, vorzugsweise 0,5 bis 2 Mol an Halogenid der Formel (IV) ein.

Als Verdünnungsmittel zur Durchführung des erfindungsgemäßen Verfahrens (d) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-tert-amylether, Dioxan, Tetrahydrofuran, 1,2-Dimethoxyethan, 1,2-Diethoxyethan oder Anisol; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, Triethylenglykol, deren Gemische mit Wasser oder reines Wasser.

Das erfindungsgemäße Verfahren (d) wird in Gegenwart eines Metalls durchgeführt. Als solche kommen vorzugsweise Übergangsmetalle, wie beispielsweise Palladium, Platin, Rhodium, Nickel, Eisen, Cobalt, Ruthenium, Iridium oder Osmium infrage. Die Metalle können gegebenenfalls an Trägermaterialien, wie z. B. Kohle, Harze, Zeolithe, Alkali- oder Erdalkalisulfate gebunden sein.

Das erfindungsgemäße Verfahren (d) wird in Gegenwart eines Reduktionsmittels durchgeführt. Als solche kommen vorzugsweise elementarer Wasserstoff, Formiatsalze, vorzugsweise Alkaliformiatsalze, wie z. B. Natriumformiat, aber auch Ammoniumformiat oder auch Metallhydride (Hydrodehalogenierung) infrage.

Das erfindungsgemäße Verfahren (d) kann in Gegenwart von Säuren durchgeführt werden. Als solche kommen vorzugsweise organische Säuren, wie z. B. Ameisensäure, Essigsäure, Ascorbinsäure, aber auch Mineralsäuren, wie z.B. Salzsäure oder Schwefelsäure infrage.

Das erfindungsgemäße Verfahren (d) kann in Gegenwart von Basen durchgeführt werden. Als solche kommen vorzugsweise organische Basen, wie z. B. Pyridin, aber auch wässrige Lösungen von Alkali- oder Erdalkalimetallhydroxiden, wie z.B. Natriumhydroxid oder Bariumhydroxid infrage.

Die Reaktionstemperaturen können bei der Durchführung des erfindungsgemäßen Verfahrens (d) in einem größeren Bereich variiert werden. Im allgemeinen arbeitet man bei Temperaturen von -80°C bis 300°C, vorzugsweise bei Temperaturen von 0°C bis 200°C.

Bei der Verwendung von elementarem Wasserstoff wird das erfindungsgemäße Verfahren (d) unter einem Wasserstoffdruck zwischen 0.5 and 200 bar, bevorzugt zwischen 1 und 100 bar durchgeführt.

Zur Durchführung des erfindungsgemäßen Verfahrens (d) zur Herstellung der Verbindungen der Formel (III-c) setzt man pro Mol des Anilin-Derivates der Formel (III-d) im Allgemeinen 0,8 bis 1000 Mol, vorzugsweise 1 bis 500 Mol an Reduktionsmittel (Ammoniumformiat, Hydrid etc.) ein.

Als Verdünnungsmittel zur Durchführung des erfindungsgemäßen Verfahrens (e) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methyl-cyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-tert-amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, Triethylenglykol, deren Gemische mit Wasser oder reines Wasser.

Das erfindungsgemäße Verfahren (e) wird in Gegenwart einer Base durchgeführt. Als solche kommen vorzugsweise Erdalkalimetall- oder Alkalimetallhydroxide, wie beispielsweise Natriumhydroxid, Kaliumhydroxid, Ammoniumhydroxid infrage.

Die Reaktionstemperaturen können bei der Durchführung des erfindungsgemäßen Verfahrens (e) in einem größeren Bereich variiert werden. Im allgemeinen arbeitet man bei Temperaturen von 100°C bis 300°C, vorzugsweise bei Temperaturen von 150°C bis 250°C.

Zur Durchführung des erfindungsgemäßen Verfahrens (e) zur Herstellung der Verbindungen der Formel (III-e) setzt man pro Mol des Haloalkanonanilins der Formel (V) im allgemeinen 0,2 bis 5 Mol, vorzugsweise 0,5 bis 3 Mol an Hydrazin oder Hydrazinhydrat ein.

Als Verdünnungsmittel zur Durchführung des erfindungsgemäßen Verfahrens (f) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-tert-amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, Triethylenglykol, deren Gemische mit Wasser oder reines Wasser.

Das erfindungsgemäße Verfahren (f) wird in Gegenwart einer Säure durchgeführt. Als solche kommen vorzugsweise Mineralsäuren, wie z. B. Salzsäure, Iod- oder Bromwasserstoffsäure, Schwefelsäure oder auch organische Säuren, z. B. Trifluoressigsäure, Trifluormethansulfonsäure infrage.

Die Reaktionstemperaturen können bei der Durchführung des erfmdungsgemäßen Verfahrens (f) in einem größeren Bereich variiert werden. Im allgemeinen arbeitet man bei Temperaturen von 0°C bis 300°C, vorzugsweise bei Temperaturen von 20°C bis 200°C.

Zur Durchführung des erfindungsgemäßen Verfahrens (f) zur Herstellung der Verbindungen der Formel (V) setzt man pro Mol des geschützten Haloalkanonanilins der Formel (VI) im Allgemeinen 0,1 bis 10000 Mol, vorzugsweise 1 bis 2000 Mol an Säure ein.

Als Verdünnungsmittel zur Durchführung des erfindungsgemäßen Verfahrens (g) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-tert-amylether, Dioxan, Tetrahydrofuran, 1,2-Dimethoxyethan, 1,2-Diethoxyethan oder Anisol;.

Das erfindungsgemäße Verfahren (g) wird in Gegenwart einer metallorganischen Verbindung durchgeführt. Als solche kommen vorzugsweise Lithiumorganische Verbindungen, wie n-, sec-, oder tert-Butyllithium, Phenyllithium oder Methyllithium infrage.

Die Reaktionstemperaturen können bei der Durchführung des erfindungsgemäßen Verfahrens (g) in einem größeren Bereich variiert werden. Im allgemeinen arbeitet man bei Temperaturen von -120°C bis 100°C, vorzugsweise bei Temperaturen von -80°C bis 20°C.

Zur Durchführung des erfindungsgemäßen Verfahrens (g) zur Herstellung der Verbindungen der Formel (VI) setzt man pro Mol des geschützten Anilins der Formel (VII) im Allgemeinen 0,2 bis 5 Mol, vorzugsweise 0,5 bis 2 Mol an Ester der Formel (VIII) ein.

Als Verdünnungsmittel zur Durchführung des erfindungsgemäßen Verfahrens (h) kommen alle inerten organischen Lösungsmittel in Betracht. Hierzu gehören vorzugsweise aliphatische oder alicyclische Kohlenwasserstoffe, wie beispielsweise Petrolether, Hexan, Heptan, Cyclohexan, Methyl-cyclohexan oder Decalin; Ether, wie Diethylether, Diisopropylether, Methyl-tert-butylether, Methyl-tert-amylether, Dioxan, Tetrahydrofuran, 1,2-Dimethoxyethan oder 1,2-Diethoxyethan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren (h) wird in Gegenwart eines Katalysators durchgeführt. Als solche kommen alle Katalysatoren infrage, die für Hydrierungen üblicherweise verwendet werden. Beispielhaft seien genannt: Raney-Nickel, Palladium oder Platin, gegebenenfalls auf einem Träger-material, wie beispielsweise Aktivkohle.

Die Hydrierung im erfindungsgemäßen Verfahren (h) kann statt in Gegenwart von Wasserstoff in Kombination mit einem Katalysator auch in Anwesenheit von Triethylsilan durchgeführt werden.

Die Reaktionstemperaturen können bei der Durchführung des erfindungsgemäßen Verfahrens (h) in einem größeren Bereich variiert werden. Im allgemeinen arbeitet man bei Temperaturen von 0°C bis 150°C, vorzugsweise bei Temperaturen von 20°C bis 100°C.

Das erfindungsgemäße Verfahren (h) wird unter einem Wasserstoffdruck zwischen 0.5 and 200 bar, bevorzugt zwischen 2 und 50 bar, besonders bevorzugt zwischen 3 und 10 bar durchgeführt.

Als Verdünnungsmittel zur Durchführung des erfindungsgemäßen Verfahrens (i) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren (i) wird gegebenenfalls in Gegenwart einer Säure durchgeführt. Als solche kommen alle anorganischen und organischen Protonen- wie auch Lewissäuren, sowie auch alle polymeren Säuren infrage. Hierzu gehören beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Titantetrachlorid, Tetrabutylorthotitanat, Zinkchlorid, Eisen-III-chlorid, Antimonpentachlorid, saure Ionenaustauscher, saure Tonerden und saures Kieselgel.

Die Reaktionstemperaturen können bei der Durchführung des erfindungsgemäßen Verfahrens (i) in einem größeren Bereich variiert werden. Im allgemeinen arbeitet man bei Temperaturen von 0°C bis 150°C, vorzugsweise bei Temperaturen von 0°C bis 80°C.

Die erfindungsgemäßen Verfahren (i) und (h) können auch in einer Tandemreaktion ("Eintopf-Reaktion") durchgeführt werden. Dazu wird eine Verbindung der Formel (X) gegebenenfalls in Gegenwart eines Verdünnungsmittels (geeignete Lösungsmittel wie für Verfahren (i)), gegebenenfalls in Gegenwart einer Säure (geeignete Säuren wie für Verfahren (i)) und in Anwesenheit von Triethylsilan umgesetzt.

Als Verdünnungsmittel zur Durchführung des erfindungsgemäßen Verfahrens (k) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-tert-amylether, Dioxan, Tetrahydrofuran, 1,2-Dimethoxyethan, 1,2-Diethoxyethan oder Anisol.

Das erfindungsgemäße Verfahren (k) wird in Gegenwart einer metallorganischen Verbindung durchgeführt. Als solche kommen vorzugsweise Methylmagnesium-chlorid, -bromid, oder -iodid oder Methyllithium infrage.

Die Reaktionstemperaturen können bei der Durchführung des erfindungsgemäßen Verfahrens (k) in einem größeren Bereich variiert werden. Im allgemeinen arbeitet man bei Temperaturen von -120°C bis 200°C, vorzugsweise bei Temperaturen von -80°C bis 100°C.

Zur Durchführung des erfindungsgemäßen Verfahrens (k) zur Herstellung der Verbindungen der Formel (X) setzt man pro Mol des Haloalkanonanilins der Formel (V) im Allgemeinen 0,8 bis 10 Mol, vorzugsweise 1 bis 5 Mol an metallorganischer Verbindung ein.

Wenn nicht anders angegeben, werden alle erfindungsgemäßen Verfahren im Allgemeinen unter Normaldruck durchgeführt. Es ist jedoch auch möglich, unter erhöhtem oder vermindertem Druck-im Allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides, Rhizoctonia-Arten, wie beispielsweise Rhizoctonia solani.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Puccinia-Arten und von Krankheiten im Wein-, Obst- und Gemüseanbau, wie beispielsweise gegen Botrytis-, Venturia- oder Alternaria-Arten, einsetzen. Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/ oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:

### Fungizide:

2-Phenylphenol; 8-Hydroxychinolinsulfät; Acibenzolar-S-methyl; Aldimoiph; Amidoflumet; Ampro-pylfos; Ampropylfos-potassium; Andoprim; Anilazine; Azaconazole; Azoxystrobin; Benalaxyl; Benalaxyl-M; Benodanil; Benomyl; Benthiavalicarb-isopropyl; Benzamacril; Benzamacril-isobutyl; Bilana-fos; Binapacryl; Biphenyl; Bitertanol; Blasticidin-S; Bromuconazole; Bupirimate; Buthiobate; Butylamin; Calcium polysulfide; Capsimycin; Captafol; Captan; Carbendazim; Carboxin; Carpropamid; Car-vone; Chinomethionat; Chlobenthiazone; Chlorfenazole; Chloroneb; Chlorothalonil; Chlozolinate; Clo-zylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenari-mol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover, Flumorph; Fluoromide; Fluoxa-strobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Imin-octadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-iso-propyl; Noviflumuron; Nuarimol; Ofurace; Orysastrobin; Oxadixyl; Oxolinic acid; Oxpoconazole; Oxy-carboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur, Pyrrolnitrine; Quinconazole; Quinoxyfen; Quintozene; Simeconazole; Spiroxamine; Sulfur; Tebuconazole; Tecloftalam; Tecnazene; Tetcyclacis; Tetra-conazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vin-clozolin; Zineb; Ziram; Zoxamide; (2S)-N-[2-[4-[[3-(4-Chlorphenyl)-2 propinyl]oxy]-3-methoxyphe-nyl]ethyl]-3-methyl-2-[(methylsulfonyl)amino]-butanamid; 1-(1-Naphthalenyl)-1H-pyrrol-2,5-dion; 2,3,5,6-Tetrachlor-4-methylsulfonyl)-pyridin; 2-Amino-4-methyl-N-phenyl-5-thiazolcarboxamid; 2-Chlor-N-(2,3-dihydro-1,1,3-trimethyl-1H-inden-4-yl)-3-pyridincarboxamide; 3,4,5-Trichlor-2,6-pyridin-dicarbonitril; Actinovate; cis-1-(4-Chlorphenyl)-2-(1H-1,2,4-triazol-1-yl)-cycloheptanol; Methyl 1-(2,3-dihydro-2,2-dimethyl-1H-inden-1-yl)-1H-imidazol-5-carboxylat; Monokaliumcarbonat; N-(6-Methoxy-3-pyridmyl)-cyclopropancarboxamid; N-Butyl-8-(1,1-dimethylethyl)-1-oxaspiro[4.5]decan-3-amin; Na-triumtehathiocarbonat; sowie Kupfersalze und -zubereitungen, wie Bordeaux mixture; Kupferhydroxid; Kupfernaphthenat; Kupferoxychlorid; Kupfersulfat; Cufraneb; Kupferoxid; Mancopper; Oxine-copper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

### 1. Acetylcholinesterase (AChE) Inhibitoren

1.1 Carbamate (z.B. Alanycarb, Aldicarb, Aldoxycarb, Allyxycarb, Aminocarb, Azamethiphos, Bendiocarb, Benfuracarb, Bufencarb, Butacarb, Butocarboxim, Butoxycarboxim, Carbaryl, Carbofuran, Carbosulfan, Chloethocarb, Coumaphos, Cyanofenphos, Cyanophos, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Triazamate, Trimethacarb, XMC, Xylylcarb)
1.2 Organophosphate (z.B. Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfen-vinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupy-razofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Para-thion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion)

### 2. Natrium-Kanal-Modulatoren / Spannungsabhängige Natrium-Kanal-Blocker

2.1 Pyrethroide (z.B. Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, DDT, Deltamethrin, Em-penthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (1R-isomer), Tralome-thrin, Transfluthrin, ZXI 8901, Pyrethrins (pyrethrum))
2.2 Oxadiazine (z.B. Indoxacarb)

### 3. Acetylcholin-Rezeptor-Agonisten/-Antagonisten

3.1 Chloronicotinyle/Neonicotinoide (z.B. Acetamiprid, Clothianidin, Dinotefuran, Imidacloprid, Nitenpyram, Nithiazine, Thiacloprid, Thiamethoxam)
3.2 Nicotine, Bensultap, Cartap

### 4. Acetylcltolin-Rezeptor-Modulatoren

4.1 Spinosyne (z.B. Spinosad)

### 5. GABA-gesteuerte Clilond-Kanal-Antagonisten

5.1 Cyclodiene Organochlorine (z.B. Camphechlor, Chlordane, Endosulfan, Gamma-HCH, HCH, Heptachlor, Lindane, Methoxychlor
5.2 Fiprole (z.B. Acetoprole, Ethiprole, Fipronil, Vaniliprole)

### 6. Chlorid-Kanal Aktivatoren

6.1 Mectine (z.B. Abamectin, Avermectin, Emamectin, Emamectin-benzoate, Ivermectin, Milbemectin, Milbemycin)

### 7. Juvenilhonnon-Mimetika

(z.B. Diofenolan, Epofenonane, Fenoxycarb, Hydroprene, Kinoprene, Methoprene, Pyriproxifen, Triprene)

### 8. Ecdysonagonistenldisruptoren

8.1 Diacylhydrazine (z.B. Chromafenozide, Halofenozide, Methoxyfenozide, Tebufenozide)

### 9. Inhibitoren der Chitinbiosynthese

9.1 Benzoylharnstoffe (z.B. Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron)
9.2 Buprofezin
9.3 Cyromazine

### 10. Inhibitoren der oxidativen Phosphorylierung, ATP-Disruptoren

10.1 Diafenthiuron
10.2 Organotine (z.B. Azocyclotin, Cyhexatin, Fenbutatin-oxide)

### 11. Entkoppler der oxidativen Phoshorylierung durch Unterbrechung des H-Protongradienten

11.1 Pyrrole (z.B. Chlorfenapyr)
11.2 Dinitrophenole (z.B. Binapacryl, Dinobuton, Dinocap, DNOC)

### 12. Seite-I-Elektronentransportinhibitoren

12.1 METI's (z.B. Fenazaquin, Fenpyroximate, Pyrimidifen, Pyridaben, Tebufenpyrad, Tolfenpyrad)
12.2 Hydramethylnone
12.3 Dicofol

### 13. Seite II Elektronentransportinhibitoren

13.1 Rotenone

### 14. Seite III Elektronentransportinhibitoren

14.1 Acequinocyl, Fluacrypyrim

### 15. Mikrobielle Disruptoren der Insektendarmmembran

Badillus thuringiensis-Stämme

### 16. Inhibitoren der Fettsynthese

16.1 Tetronsäuren (z.B. Spirodiclofen, Spiromesifen)
16.2 Tetramsäuren [z.B. 3-(2,5-Dimethylphenyl)-8-methoxy-2-oxo-1-azaspiro[4.5]dec-3-en-4-yl ethyl carbonate (alias: Carbonic acid, 3-(2,5-dimethylphenyl)-8-methoxy-2-oxo-1-azaspiro[4.5]dec-3-en-4-yl ethyl ester, CAS-Reg.-No.: 382608-10-8) and Carbonic acid, cis-3-(2,5-dimethylphenyl)-8-methoxy-2-oxo-1-azaspiro[4.5]dec-3-en-4-yl ethyl ester (CAS-Reg.-No.: 203313-25-1)]

### 17. Carboxamide

(z.B. Flonicamid)

### 18. Oktopaminerge Agonisten

(z.B. Amitraz)

### 19. Inhibitoren der Magnesium-stimulierten ATPase

(z.B. Propargite)

### 20. Phthalamide

(z.B. N²-[1,1-Dimethyl-2-(methylsulfonyl)ethyl]-3-iod-N¹-[2-methyl-4-[1,2,2,2-tetrafluor-1-(trifluor-methyl)ethyl]phenyl]-1,2-benzenedicarboxamide (CAS-Reg.-No.: 272451-65-7), Flubendiamide)

### 21. Nereistoxin-Analoge

(z.B. Thiocyclam hydrogen oxalate, Thiosultap-sodium)

### 22. Biologika, Hormone oder Pheromone

(z.B. Azadirachtin, Bacillus spec., Beauveria spec., Codlemone, Metarrhizium spec., Paecilomyces spec., Thuringiensin, Verticillium spec.)

### 23. Wirkstoffe mit unbekannten oder nicht spezifischen Wirkmechanismen

23.1 Begasungsmittel (z.B. Aluminium phosphide, Methyl bromide, Sulfuryl fluoride)
23.2 Selektive Fraßhemmer (z.B. Cryolite, Flonicamid, Pymetrozine)
23.3 Milbenwachstumsinhibitoren (z.B. Clofentezine, Etoxazole, Hexythiazox)
23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflunletofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyrafluprole, Pyridalyl, Pyriprole, Sulfluramid, Tetradifon, Tetrasul, Triarathene, Verbutin,
ferner die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernähruxigswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfmdungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigen-schaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirk-stoffen, z.B. Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kom-binationen miteinander in den transgenen Pflanzen vorkommen. Als Beispiele fiir "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link@ (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Beispiel 2

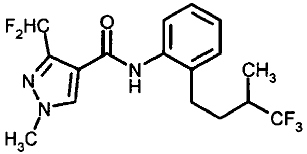

Zu einer Suspension von 264.2 mg (1.5 mmol) 3-Difluormethyl-1-methyl-1H-pyrazol-4-carbonsäure in 9 ml Dichlormethan werden 0.14 ml (1.7 mmol) Oxalsäuredichlorid und 4 Tropfen Dimethylformamid gegeben. Die Reaktionsmischung wird 2 h bei Raumtemperatur gerührt und anschließend mit einer Lösung bestehend aus 325.9 mg (1.5 mmol) 2-(4,4,4-Trifluor-3-methyl-butyl)-phenylamin und 0.29 ml (2.1 mmol) Triethylamin in 9 ml Dichlormethan versetzt. Die Reaktionsmischung wird 16 h bei Raumtemperatur gerührt. Zur Aufarbeitung werden 7 ml 2N Salzsäure zugegeben, 10 min bei Raumtemperatur gerührt, die organische Phase abgetrennt, über Magnesiumsulfat getrocknet, filtriert und im Vakuum aufkonzentriert.

Man erhält 525.0 mg (89 % der Theorie) an 3-Difluormethyl-1-methyl-1H-pyrazol-4-carbonsäure-[2-(4,4,4-trifluor-3-methyl-butyl)-phenyl]-amid [logP (pH 2.3) = 2.93].

Analog Beispiel 1 und 2, sowie entsprechend den Angaben in der allgemeinen Beschreibung der erfindungsgemäßen Herstellverfahren (a) bis (h) wurden auch die in der nachstehenden Tabelle 1 genannten Verbindungen der Formel (I) erhalten:

**Tabelle 1**

| | | | | | | | | |
|---|---|---|---|---|---|---|---|---|
| 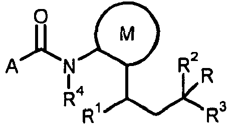 | | | | | | **(I)** | | |

| **Nr.** | **R** | **R¹** | **R²** | **R³** | **R⁴** | **M** | **A** | **logP** |
|---|---|---|---|---|---|---|---|---|
| 3 | H | CH₃ | C₂H₅ | Cl | H | 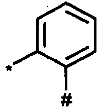 | 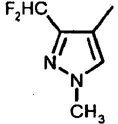 | 3,18 |
| 4 | H | CH₃ | C₂H₅ | Cl | H | 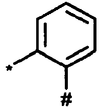 | 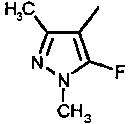 | 3,09 |
| 6 | H | H | CH₃ | CF₃ | H | 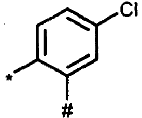 | 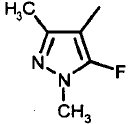 | 3,32 |
| 7 | H | H | CH₃ | CF₃ | H | 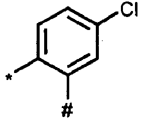 | 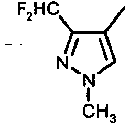 | 3,36 |
| 10 | H | H | CH₃ | CF₃ | H | 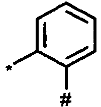 | 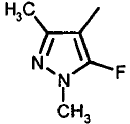 | 2,84 |

| | | | | | | | | |
|---|---|---|---|---|---|---|---|---|
| ^{a}) Die mit "*" markierte Bindung ist mit dem Amid, die mit "#" markierte Bindung mit dem Haloalkylrest verknüpft. | | | | | | | | |

### Herstellung der Ausgangsstoffe der Formel (III)

### Beispiel (III-1)

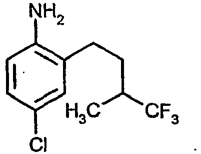

Eine Lösung von 7.0 g 1-(2-Amino-5-chlor-phenyl)-4,4,4-trifluor-3-methyl-butan-1-on (26 mmol), 4.0 g Kaliumhydroxid (60 mmol) und 3.0 g Hydrazinhydrat (60 mmol) wird in 67 ml Triethylenglykol für 6 h auf 210°C erhitzt. Nach Abkühlen auf Raumtemperatur werden Wasser und Ethylacetat zugegeben, die Phasen getrennt und die organische Phase nochmals mit Wasser gewaschen, über Magnesiumsulfat getrocknet und vom Lösungsmittel befreit.

Man erhält 4.9 g (73 % der Theorie) an 4-Chlor-2-(4,4,4-trifluor-3-methyl-butyl)-phenylamin.

¹H-NMR (DMSO): δ = 6.93 (m, 2 H), 6.62 (d, 1H), 5.05 (s, 2 H), 2.56 (m, 1 H), 2.48-2.34 (m, 2 H), 1.85 (m, 1H), 1.47 (m, 1 H), 1.11 (d, 3 H).

### Beispiel (III-2)

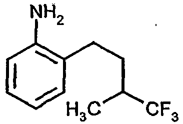

Eine Lösung von 2.3 g 4-Chlor-2-(4,4,4-trifluor-3-methyl-butyl)-phenylamin (III-1) (9 mmol), 1.15 g Ammoniumformiat (18 mmol) und 2.0 g Pd/C (5%ig, 0.9 mmol) in 21 ml Methanol wird 1 h bei Raumtemperatur gerührt. Anschließend wird die Reaktionslösung über Celite abgesaugt, mit Methanol nachgewaschen und das Filtrat einrotiert. Verrühren des Rückstandes nach Entfernen des Lösungsmittels mit Pentan liefert einen Feststoff, der abgesaugt und getrocknet wird.

Man erhält 1.7 g (86 % der Theorie) an 2-(4,4,4-Trifluor-3-methyl-butyl)-phenylamin.
¹H-NMR (DMSO): δ = 6.89 (m, 2 H), 6.61 (m, 1H), 6.49 (m, 1 H), 4.83 (s, 2 H), 2.57 (m, 1H), 2.48-2.30 (m, 2 H), 1.85 (m, 1 H), 1.46 (m, 1 H), 1.13 (d, 3 H).

### Herstellung von Auseagesstoffen der Formel (V)

### Beispiel (V-1)

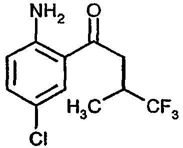

Eine Lösung von 10.0 g (29 mmol) N-[4-Chlor-2-(4,4,4-trifluor-3-methyl-butyryl)-phenyl]-2,2-dimethyl-propionamid in 366 ml 37%iger Salzsäure wird 2 Tage unter Rückfluss erhitzt. Nach Abkühlen auf Raumtemperatur wird mit 45%iger Natriumhydroxid-Lösung neutralisiert und die Wasserphase mit Dichlormethan extrahiert. Trocknen der organischen Phase über Natriumsulfat und Entfernen des Lösungsmittels liefert 7.1 g (93 % der Theorie) an 1-(2-Amino-5-chlor-phenyl)-4,4,4-trifluor-3-methyl-butan-1-on.

¹H-NMR (DMSO): δ = 7.80 (d, 1 H), 7.34 (s, 2 H), 7.29 (dd, 1 H), 6.82 (dd, 1 H), 3.28 (dd, 1 H), 3.14 (dd, 1 H) 3.00 (m, 1 H), 1.08 (d, 3 H).

### Herstellung von Ausgangsstoffen der Formel (VI)

### Beispiel (VI-1)

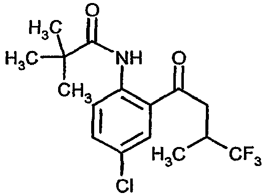

Eine Lösung von 15.4 g N-(4-Chlorphenyl) 2,2-dimethyl-propionamid (73 mmol) in 100 ml trockenem Tetrahydrofuran wird bei 0°C tropfenweise mit einer Lösung von n-Butyllithium in Hexan (1.6 M, 100 ml, 160 mmol) versetzt und 2 h bei dieser Temperatur gerührt. Anschließend wird diese Lösung bei -70°C zu einer Lösung von 13.4 g Ethyl-(3-trifluormethyl)-butyrat (73 mmol) in 250 ml trockenem Tetrahydrofuran zugetropft und die Reaktionsmischung 1 h bei dieser Temperatur nachgerührt. Nach Erwärmen auf Raumtemperatur wird für 16 h nachgerührt. Hydrolyse mit 100 ml Wasser, Einengen, Aufnehmen des Rückstandes in Dichlormethan/Wasser sowie Extrahieren der Wasserphase mit Dichlormethan liefert nach Trocknen der organischen Phase über Natriumsulfat ein Edukt/ Produkt-Gemisch, welches durch Säulenchromatographie an Kieselgel mit Cyclohexan/Essigsäureethylester (9:1) als Laufmittel aufgetrennt werden kann.

Man erhält 10.3 g (40 % der Theorie) an N-[4-Chlor-2-(4,4,4-trifluor-3-methyl-butyryl)-phenyl]-2,2-dimethyl-propionamid.

¹H-NMR (DMSO): δ =11.15 (s, 1 H), 8.39 (d, 1 H), 8.11 (d, 1 H), 7.67 (dd, 1 H), 3.47 (dd, 1 H), 3.30 (dd, 1 H) 2.99 (m, 1 H), 1.25 (s, 9 H), 1.15 (d, 3 H).

Die Bestimmung der angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.

Eluenten für die Bestimmung im sauren Bereich (pH 2,3): 0,1 % wässrige Phosphorsäure, Acetonitril; linearer Gradient von 10 % Acetonitril bis 90 % Acetonitril.

Die Eichung erfolgte mit unverzweigten Alkan-2-onen (mit 3 bis 16 Kohlenstoffatomen), deren LogP-Werte bekannt sind (Bestimmung der LogP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Sphaerotheca-Test (Gurke) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von Sphaerotheca fuliginea inokuliert. Die Pflanzen werden dann bei ca. 23°C und einer relativen Luftfeuchtigkeit von ca. 70 % im Gewächshaus aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle A**

| **Sphaerotheca-Test (Gurke) / protektiv** | | |
|---|---|---|
| Wirkstoff Erfindungsgemäß | Aufwandmenge an Wirkstoff in g/ha | Wirkungsgrad in % |
| 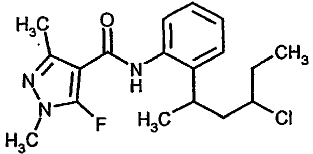 | 100 | 95 |
| 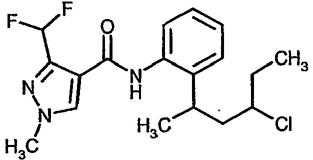 | 100 | 100 |
| 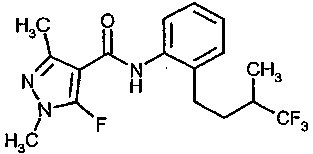 | 100 | 100 |
| 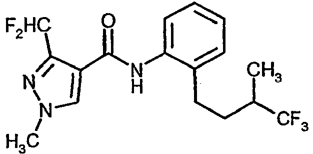 | 100 | 98 |

### Beispiel B

### Venturia - Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle B**

| **Venturia-Test (Apfel) / protektiv** | | |
|---|---|---|
| Wirkstoff Erfindungsgemäß | Aufwandmenge an Wirkstoff in g/ha | Wirkungsgrad in % |
| 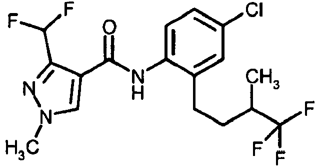 | 100 | 93 |
| 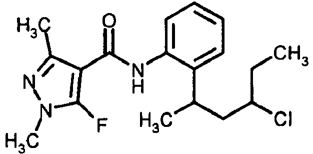 | 100 | 100 |
| 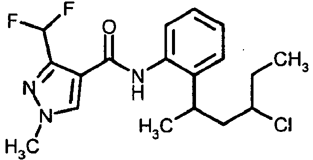 | 100 | 100 |
| 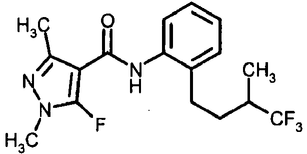 | 100 | 94 |
| 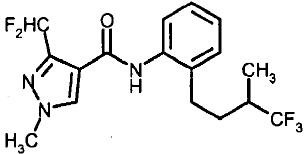 | 100 | 97 |

### Beispiel C

### Botrytis - Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten Kammer bei ca. 20°C und 100 % relativer Luftfeuchtigkeit aufgestellt

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

**Tabelle C**

| **Botrytis - Test (Bohne) / protektiv** | | |
|---|---|---|
| Wirkstoff Erfindungsgemäß | Aufwandmenge an Wirkstoff in g/ha | Wirkungsgrad in % |
| 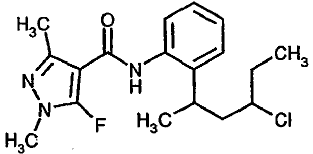 | 500 | 82 |
| 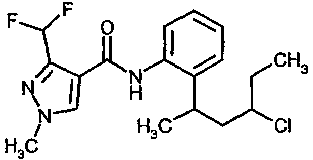 | 500 | 100 |
| 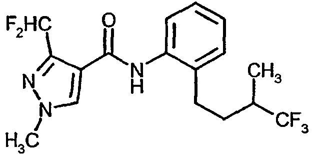 | 500 | 99 |

### Beispiel D

### Puccinia-Test (Weizen) / protektiv

| | | |
|---|---|---|
| Lösungsmittel: | 50 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle D**

| **Puccinia-Test (Weizen) / protektiv** | | |
|---|---|---|
| Wirkstoff Erfindungsgemäß | Aufwandmenge an Wirkstoff in g/ha | Wirkungsgrad in % |
| 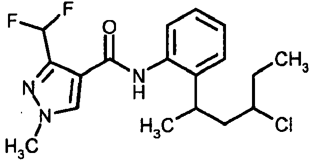 | 500 | 100 |
| 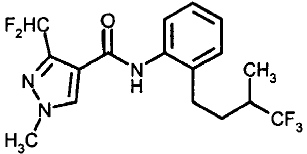 | 500 | 100 |

### Beispiel E

### Alternaria-Test (Tomate) / protektiv

| | | |
|---|---|---|
| Lösungsmittel: | 49 | Gewichtsteile N, N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von Alternaria solani inokuliert und stehen dann 24 h bei 100 % rel. Feuchte und 20°C. Anschließend stehen die Pflanzen bei 96 % rel. Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle E**

| **Alternaria-Test (Tomate) / protektiv** | | |
|---|---|---|
| Wirkstoff Erfindungsgemäß | Aufwandmenge an Wirkstoff in g/ha | Wirkungsgrad in % |
| 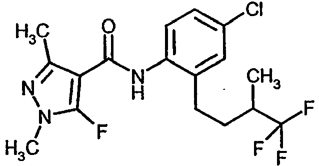 | 750 | 90 |
| 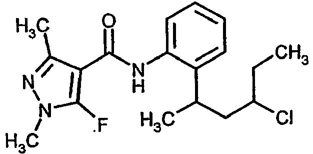 | 750 | 100 |
| 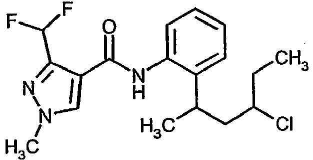 | 750 | 95 |

## Patentansprüche

1. Haloalkylcarboxamide der Formel (I) 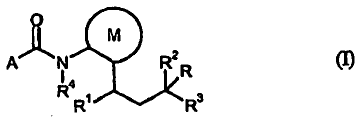 in welcher
R für Wasserstoff oder Halogen steht,
R¹ für Wasserstoff oder Methyl steht,
R² für Methyl, Ethyl oder C₁-C₄-Halogenalkyl mit 1 bis 9 Fluor-, Chlor-und/oder Brom-atomen steht,
R³ für Halogen oder C₁-C₄-Halogenalkyl mit 1 bis 9 Fluor-, Chlor- und/oder Brom-atomen steht,
R⁴ für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)Jcarbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₈-Halogencycloalkyl)carbonyl mit jeweils 1 bis 9 Fluor-, Chlor- und/oder Bromatomen; oder -C(=O)C(=O)R⁵, -CONR⁶R⁷ oder -CH₂NR⁸R⁹ steht,
R⁵ für Wasserstoff C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
R⁶ und R⁷ unabhängig voneinander jeweils für Wasserstoff C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
R⁶ und R⁷ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis. 8 Ringatomen bilden, wobei der Heterocyclus 1 oder 2 weitere, nicht benachbarte Heteroatome aus der Reihe Sauerstoff, Schwefel oder NR¹⁰ enthalten kann,
R⁸ und R⁹ unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
R⁸ und R⁹ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹⁰ enthalten kann,
R¹⁰ für Wasserstoff oder C₁-C₆-Alkyl steht,
M für einen jeweils einfach durch R¹¹ substituierten Phenyl-,
R¹¹ für Wasserstoff, Fluor, Chlor, Methyl, iso-Propyl, Methylthio oder Trifluormethyl steht,
A für den Rest der Formel (A1) 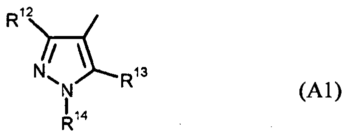 steht, in welcher
R¹² für Wasserstoff, Cyano, Halogen, Nitro, C₁-C₄-Alkyl, C₁-C₄-Alkoxy, C₁-C₄-Alkylthio, C₃-C₆-Cycloalkyl, C₁-C₄-Halogenalkyl, C₁-C₄-Halogenalkoxy oder C₁-C₄-Halogenalkylthio mit jeweils 1 bis 5 Halogenatomen, Aminocarbonyl oder Aminocarbonyl-C₁-C₄-alkyl steht,
R¹³ für Wasserstoff, Halogen, Cyano, C₁-C₄-Alkyl, C₁-C₄-Alkoxy oder C₁-C₄-Alkylthio steht,
R¹⁴ für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder Phenyl steht,

2. Haloälkylcarboxamide der Formel (I) gemäß Anspruch 1, in welcher
R für Wasserstoff Fluor, Chlor oder Brom steht,
R¹ für Wasserstoff oder Methyl steht,
R² für Methyl, Ethyl oder für jeweils einfach oder mehrfach, gleich oder verschieden durch Fluor, Chlor oder Brom substituiertes Methyl, Ethyl, n- oder iso-Propyl, n-, iso-, sec- oder tert-Butyl steht,
R³ für Fluor, Chlor, Brom, Iod oder für jeweils einfach oder mehrfach, gleich oder verschieden durch Fluor, Chlor oder Brom substituiertes Methyl, Ethyl, n- oder iso-Propyl, n-, iso-, sec- oder tert-Butyl steht,
R⁴ für Wasserstoff, C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₆-Alkyl)carbonyl, (C₁-C₄-Alkoxy)carbonyl, (C₁-C₃-Alkoxy-C₁-C₃-alkyl)carbonyl, (C₃-C₆-Cycloalkyl)carbonyl; (C₁-C₄-Halogenalkyl)carbonyl, (C₁-C₄-Halogenalkoxy)carbonyl, (Halogen-C₁-C₃-alkoxy-C₁-C₃-alkyl)carbonyl, (C₃-C₆-Halogencycloalkyl)carbonyl mit jeweils 1 bis 9 Fluor-, Chlor- und/oder Bromatomen; oder -C(=O)C(=O)R⁵, -CONR⁶R⁷ oder -CH₂NR⁸R⁹ steht,
R⁵ für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Brom-atomen steht,
R⁶ und R⁷ unabhängig voneinander jeweils für Wasserstoff, C₁-C₆-Alkyl, C₁-C₃-Alkoxy-C₁-C₃-alkyl, C₃-C₆-Cycloalkyl; C₁-C₄-Halogenalkyl, Halogen-C₁-C₃-alkoxy-C₁-C₃-alkyl, C₃-C₆-Halogencycloalkyl mit jeweils 1 bis 9 Fluor-, Chlor- und/oder Bromatomen stehen,
R⁶ und R⁷ außerdem gemeinsam mit dem Stickstoffatom, an das sie gebunden sind, einen gegebenenfalls einfach bis vierfach, gleich oder verschieden durch Halogen oder C₁-C₄-Alkyl substituierten gesättigten Heterocyclus mit 5 oder 6 Ringatomen bilden, wobei der Heterocyclus 1 oder 2 weitere, nicht benachbarte Heteroatome aus der Reihe Sauerstoff, Schwefel oder NR¹⁰ enthalten kann,
R⁸ und R⁹ unabhängig voneinander für Wasserstoff, C₁-C₆-Alkyl, C₃-C₆-Cycloalkyl; C₁-C₄-Halogenalkyl, C₃-C₆-Halogencycloalkyl mit jeweils 1 bis 9 Fluor-, Chlor- und/oder Bromatomen stehen,
R⁸ und R⁹ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 oder 6 Ringatomen bilden, wobei der Heterocyclus 1 oder 2 weitere, nicht benachbarte Heteroatome aus der Reihe Sauerstoff, Schwefel oder NR¹⁰ enthalten kann,
R¹⁰ für Wasserstoff oder C₁-C₄-Alkyl steht,
M für einen der folgenden Cyclen steht 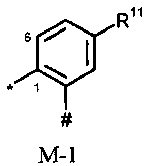 wobei die mit "*" markierte Bindung mit dem Amid, die mit "#" markierte Bindung mit dem Haloalkylrest verknüpft ist,
R¹¹ für Wasserstoff, Fluor, Chlor, Methyl oder Trifluormethyl steht,
A für den Rest der Formel (A1) 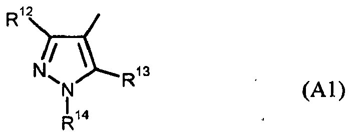 steht, in welcher
R¹² für Wasserstoff, Cyano, Fluor, Chlor, Brom, Iod, Methyl, Ethyl, iso-Propyl, Methoxy, Ethoxy, Methylthio, Ethylthio, Cyclopropyl, C₁-C₂-Halogenalkyl, C₁-C₂-Halogenalkoxy mit jeweils 1 bis 5 Fluor, Chlor und/oder Bromatomen, Trifluormethylthio, Difluormethylthio, Aminocarbonyl, Aminocarbonylmethyl oder Aminocarbonylethyl steht,
R¹³ für Wasserstoff Fluor, Chlor, Brom, Iod, Methyl, Ethyl, Methoxy, Ethoxy, Methylthio oder Ethylthio steht,
R¹⁴ für Wasserstoff Methyl, Ethyl, n-Propyl, iso-Propyl, C₁-C₂-Halogenalkyl mit 1 bis 5 Fluor, Chlor und/oder Bromatomen, Hydroxymethyl, Hydroxyethyl, Cyclopropyl, Cyclopentyl, Cyclohexyl oder Phenyl steht

3. Verfahren zum Herstellen der Haloalkylcarboxamide der Formel (I) gemäß Anspruch 1, **dadurch gekennzeichnet, dass** man
a) Carbonsäure-Derivate der Formel (II) 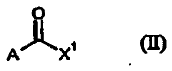 in welcher
A die in Anspruch 1 angegebenen Bedeutungen hat und
X¹ für Halogen oder Hydroxy steht, mit Anilin-Derivaten der Formel (III) 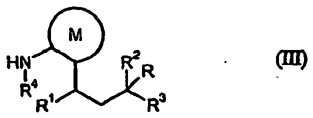
in welcher
R, R¹, R², R³, R⁴ und M die in Anspruch 1 angegebenen Bedeutungen haben, gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt,
oder
b) Hexylcarboxanilide der Formel (I-a) 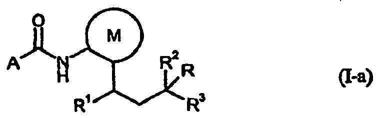 in welcher
R, R¹, R², R³, M und A die in Anspruch 1 angegebenen Bedeutungen haben mit Halogeniden der Formel (IV)
R^{4-A}-X² (IV)
in welcher
X² für Chlor, Brom oder Iod steht,
R^{4-A} für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)-carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor-und/oder Bromatomen;
(C₁-C₈-Alkyl)carbonyl, (C₁-C₈-Alkoxy)carbonyl, (C₁-C₄-Alkoxy-C₁-C₄-alkyl)-carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₈-Halogencycloakyl)carbonyl mit jeweils 1 bis 9 Fluor-, Chlor- und/oder Bromatomen; oder -C(=O)C(=O)R⁵, -CONR⁶R⁷oder -CH₂NR⁸R⁹ steht, wobei R⁵, R⁶, R⁷, R⁸ und R⁹ die in Anspruch 1 angegebenen Bedeutungen haben,
in Gegenwart einer Base und in Gegenwart eines Verdünnungsmittels umsetzt.

4. Mittel zur Bekämpfung unerwünschter Mikroorganismen, **gekennzeichnet durch** einen Gehalt an mindestens einem Haloalkylcarboxamid der Formel (I) gemäß Anspruch 1 neben Streckmitteln und/oder oberflächenaktiven Stoffen.

5. Verwendung von Haloalkylcarboxamiden der Formel (I) gemäß Anspruch 1 zur Bekämpfung unerwünschter Mikroorganismen.

6. Verfahren zur Bekämpfung unerwünschter Mikroorganismen, **dadurch gekennzeichnet, dass** man Haloalkylcarboxamide der Formel (I) gemäß Anspruch 1 auf die Mikroorganismen und/oder deren Lebensraum ausbringt.

7. Verfahren zur Herstellung von Mitteln zur Bekämpfung unerwünschter Mikroorganismen, **dadurch gekennzeichnet, dass** man Haloalkylcarboxamide der Formel (I) gemäß Anspruch 1 mit Streckmitteln und/oder oberflächenaktiven Stoffen vermischt.

## Claims

1. Haloalkyl carboxamides of the formula (I) 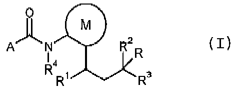 in which
R stands for hydrogen or halogen,
R¹ stands for hydrogen or methyl,
R² stands for methyl, ethyl or C₁-C₄ haloalkyl with 1 to 9 fluorine, chlorine and/or bromine atoms,
R³ stands for halogen or C₁-C₄ haloalkyl with 1 to 9 fluorine, chlorine and/or bromine atoms,
R⁴ stands for hydrogen, C₁-C₈ alkyl, C₁-C₆ alkylsulfinyl, C₁-C₆ alkylsulfonyl, C₁-C₄-alkoxy-C₁-C₄-alkyl, C₃-C₈ cycloalkyl; C₁-C₆ haloalkyl, C₁-C₄ haloalkylthio, C₁-C₄ haloalkylsulfinyl, C₁-C₄ haloalkylsulfonyl, halo-C₁-C₄-alkoxy-C₁-C₄-alkyl, C₃-C₈ halocycloalkyl with 1 to 9 fluorine, chlorine and/or bromine atoms in each case; formyl, formyl-C₁-C₃-alkyl, (C₁-C₃ alkyl)carbonyl-C₁-C₃-alkyl, (C₁-C₃ alkoxy)carbonyl-C₁-C₃-alkyl; halo-(C₁-C₃ alkyl)carbonyl-C₁-C₃-alkyl, halo-(C₁-C₃ alkoxy)carbonyl-C₁-C₃-alkyl with 1 to 13 fluorine, chlorine and/or bromine atoms in each case;
(C₁-C₈ alkyl)carbonyl, (C₁-C₈ alkoxy)carbonyl, (C₁-C₄-alkoxy-C₁-C₄-alkyl)carbonyl, (C₃-C₈ cycloalkyl)carbonyl; (C₁-C₆ haloalkyl)carbonyl, (C₁-C₆ haloalkoxy)carbonyl, (halo-C₁-C₄-alkoxy-C₁-C₄-alkyl)carbonyl, (C₃-C₈ halocycloalkyl)carbonyl with 1 to 9 fluorine, chlorine and/or bromine atoms in each case; or -C((=O)C(=O)R⁵, -CONR⁶R⁷ or -CH₂NR⁸R⁹,
R⁵ stands for hydrogen, C₁-C₈ alkyl, C₁-C₈ alkoxy, C₁-C₄-alkoxy-C₁-C₄-alkyl, C₃-C₈ cycloalkyl; C₁-C₆ haloalkyl, C₁-C₆ haloalkoxy, halo-C₁-C₄-alkoxy-C₁-C₄-alkyl, C₃-C₈ halocycloalkyl with 1 to 9 fluorine, chlorine and/or bromine atoms in each case,
R⁶and R⁷ stand independently of one another in each case for hydrogen, C₁-C₈ alkyl, C₁-C₄-alkoxy-C₁-C₄-alkyl, C₃-C₈ cycloalkyl; C₁-C₈ haloalkyl, halo-C₁-C₄-alkoxy-C₁-C₄-alkyl, C₃-C₈ halocycloalkyl with 1 to 9 fluorine, chlorine and/or bromine atoms in each case,
R⁶ and R⁷, moreover, form a substituted, saturated heterocycle with 5 to 8 ring atoms together with the nitrogen atom to which they are bound, with single or multiple, the same or various substitution by halogen or C₁-C₄ alkyl, whereby the heterocycle can contain 1 or 2 additional, non-adjacent hetero atoms constituted by oxygen, sulfur or NR¹⁰,
R⁸ and R⁹ stand independently of one another for hydrogen, C₁-C₈ alkyl, C₃-C₈ cycloalkyl; C₁-C₈ haloalkyl, C₃-C₈ halocycloalkyl with 1 to 9 fluorine, chlorine and/or bromine atoms in each case,
R⁸ and R⁹, moreover, form a substituted, saturated heterocycle with 5 to 8 ring atoms together with the nitrogen atom to which they are bound, with single or multiple, the same or various substitution by halogen or C₁-C₄ alkyl, whereby the heterocycle can contain 1 or 2 additional, non-adjacent hetero atoms constituted by oxygen, sulfur or NR¹⁰,
R¹⁰ stands for hydrogen or C₁-C₆ alkyl,
M stands for a phenyl in each case with a single substitution by R¹¹,
R¹¹ stands for hydrogen, fluorine, chlorine, methyl, isopropyl, methylthio or trifluoromethyl,
A stands for the radical of the formula (A1) 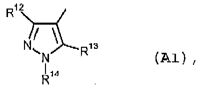 in which
R¹² stands for hydrogen, cyano, halogen, nitro, C₁-C₄ alkyl, C₁-C₄ alkoxy, C₁-C₄ alkylthio, C₃-C₆ cycloalkyl, C₁-C₄ haloalkyl, C₁-C₄ haloalkoxy or C₁-C₄ haloalkylthio, in each case with 1 to 5 halogen atoms, aminocarbonyl or aminocarbonyl-C₁-C₄-alkyl,
R¹³ stands for hydrogen, halogen, cyano, C₁-C₄ alkyl, C₁-C₄ alkoxy or C₁-C₄ alkylthio,
R¹⁴ stands for hydrogen, C₁-C₄ alkyl, hydroxy-C₁-C₄ alkyl, C₂-C₆ alkenyl, C₃-C₆ cycloalkyl, C₁-C₄-alkylthio-C₁-C₄-alkyl, C₁-C₄-alkoxy-C₁-C₄-alkyl, C₁-C₄ haloalkyl, C₁-C₄-haloalkylthio-C₁-C₄-alkyl, C₁-C₄-haloalkoxy-C₁-C₄-alkyl in each case with 1 to 5 halogen atoms, or phenyl.

2. Haloalkyl carboxamides of the formula (I) according to Claim 1, in which
R stands for hydrogen, fluorine, chlorine or bromine,
R¹ stands for hydrogen or methyl,
R² stands for methyl, ethyl or for methyl, ethyl, n- or isopropyl, n-, iso-, see or tert-butyl in each case with single or multiple, the same or various, substitution by fluorine, chlorine or bromine.
R³ stands for fluorine, chlorine, bromine, iodine or for methyl, ethyl, n- or isopropyl, n-, iso-, sec or tert-butyl in each case with single or multiple, the same or various, substitution by fluorine, chlorine or bromine.
R⁴ stands for hydrogen, C₁-C₆ alkyl, C₁-C₄ alkylsulfinyl, C₁-C₄ alkylsulfonyl, C₁-C₃-alkoxy-C₁-C₃-alkyl, C₃-C₆ cycloalkyl; C₁-C₄ haloalkyl, C₁-C₄ haloalkylthio, C₁-C₄ haloalkylsulfinyl, C₁-C₄ haloalkylsulfonyl, halo-C₁-C₃-alkoxy-C₁-C₃-alkyl, C₃-C₈ halocycloalkyl with 1 to 9 fluorine, chlorine and/or bromine atoms in each case; formyl, formyl-C₁-C₃-alkyl, (C₁-C₃ alkyl)carbonyl-C₁-C₃-alkyl, (C₁-C₃ alkoxy)carbonyl-C₁-C₃-alkyl; halo-(C₁-C₃ alkyl)carbonyl-C₁-C₃-alkyl, halo-(C₁-C₃ alkoxy)carbonyl-C₁-C₃-alkyl with 1 to 13 fluorine, chlorine and/or bromine atoms in each case;
(C₁-C₆ alkyl)carbonyl, (C₁-C₄ alkoxy)carbonyl, (C₁-C₃-alkoxy-C₁-C₃-alkyl)carbonyl, (C₃-C₆ cycloalkyl)carbonyl; (C₁-C₄ haloalkyl)carbonyl, (C₁-C₄ haloalkoxy)carbonyl, (halo-C₁-C₃-alkoxy-C₁-C₃-alkyl)carbonyl, (C₃-C₆ halocycloalkyl)carbonyl with 1 to 9 fluorine, chlorine and/or bromine atoms in each case; or -C(=O)C(=O)R⁵, -CONR⁶R⁷ or -CH₂NR⁸R⁹,
R⁵ stands for hydrogen, C₁-C₆ alkyl, C₁-C₄ alkoxy, C₁-C₃-alkoxy-C₁-C₃-alkyl, C₃-C₆ cycloalkyl; C₁-C₄ haloalkyl, C₁-C₄ haloalkoxy, halo-C₁-C₃-alkoxy-C₁-C₃-alkyl, C₃-C₆ halocycloalkyl with 1 to 9 fluorine, chlorine and/or bromine atoms in each case,
R⁶and R⁷ stand independently of one another in each case for hydrogen, C₁-C₆ alkyl, C₁-C₃-alkoxy-C₁-C₃-alkyl, C₃-C₆ cycloalkyl; C₁-C₄ haloalkyl, halo-C₁-C₃-alkoxy-C₁-C₃-alkyl, C₃-C₆ halocycloalkyl with 1 to 9 fluorine, chlorine and/or bromine atoms in each case,
R⁶ and R⁷, moreover, form a substituted, saturated heterocycle with 5 or 6 ring atoms together with the nitrogen atom to which they are bound, with single to quadruple, the same or various substitution by halogen or C₁-C₄ alkyl, whereby the heterocycle can contain 1 or 2 additional, non-adjacent hetero atoms constituted by oxygen, sulfur or NR¹⁰,
R⁸ and R⁹ stand independently of one another for hydrogen, C₁-C₆ alkyl, C₃-C₆ cycloalkyl; C₁-C₄ haloalkyl, C₃-C₆ halocycloalkyl with 1 to 9 fluorine, chlorine and/or bromine atoms in each case,
R⁸ and R⁹, moreover, form a substituted, saturated heterocycle with 5 or 6 ring atoms together with the nitrogen atom to which they are bound, with single or multiple, the same or various substitution by halogen or C₁-C₄ alkyl, whereby the heterocycle can contain 1 or 2 additional, non-adjacent hetero atoms constituted by oxygen, sulfur or NR¹⁰,
R¹⁰ stands for hydrogen or C₁-C₄ alkyl,
M stands for one of the following cycles 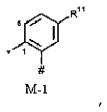 whereby the bond marked with "*" is a link with the amide, and the bond marked with "#" is a link with the haloalkyl radical,
R¹¹ stands for hydrogen, fluorine, chlorine, methyl or trifluoromethyl,
A stands for the radical of the formula (Al) 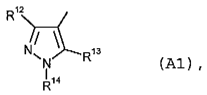 in which
R¹² stands for hydrogen, cyano, fluorine, chlorine, bromine, iodine, methyl, ethyl, isopropyl, methoxy, ethoxy, methylthio, ethylthio, cyclopropyl, C₁-C₂ haloalkyl, C₁-C₂ haloalkoxy in each case with 1 to 5 fluorine, chlorine and/or bromine atoms, trifluoromethylthio, difluoromethylthio, aminocarbonyl, aminocarbonylmethyl or aminocarbonylethyl,
R¹³ stands for hydrogen, fluorine, chlorine, bromine, iodine, methyl, ethyl, methoxy, ethoxy, methylthio or ethylthio,
R¹⁴ stands for hydrogen, methyl, ethyl, n-propel, isopropyl, C₁-C₂ haloalkyl with 1 to 5 fluorine, chlorine and/or bromine atoms, hydroxymethyl, hydroxyethyl, cyclopropyl, cyclopentyl, cyclohexyl or phenyl.

3. A process for synthesizing haloalkyl carboxamides of the formula (I) according to Claim 1, **characterized in that**
a) carboxylic acid derivatives the formula (II) 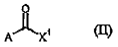 in which
A has the meanings specified in Claim 1 and
X¹ stands for halogen or hydroxy,
are reacted with aniline derivatives of the formula (III) 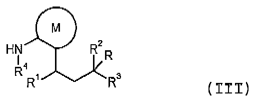 in which
R, R¹, R², R³, R⁴ and M have the meanings specified in Claim 1,
possibly in the presence of a catalyst, possibly in the presence a condensation agent, possibly in the presence of an acid binder and possibly in the presence of a diluent,
or
b) hexylcarboxanilides of the formula (I-a) 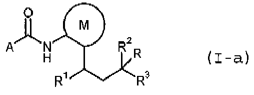 in which
R, R¹, R², R³, M and A have the meanings specified in Claim 1,
are reacted with halides of the formula (IV) 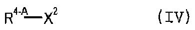 in which
X² stands for chlorine, bromine or iodine,
R^{4-A} stands for C₁-C₈ alkyl, C₁-C₆ alkylsulfinyl, C₁-C₆ alkylsulfonyl, C₁-C₄-alkoxy-C₁-C₄ alkyl, C₃-C₈ cycloalkyl; C₁-C₆ haloalkyl, C₁-C₄ haloalkylthio, C₁-C₄ haloalkylsulfinyl, C₁-C₄ haloalkylsulfonyl, halo-C₁-C₄-alkoxy-C₁-C₄-alkyl, C₃-C₈ halocycloalkyl with 1 to 9 fluorine, chlorine and/or bromine atoms in each case; formyl, formyl-C₁-C₃-alkyl, (C₁-C₃ alkyl)carbonyl-C₁-C₃-alkyl, (C₁-C₃ alkoxy)carbonyl-C₁-C₃-alkyl; halo-(C₁-C₃ alkyl)carbonyl-C₁-C₃-alkyl, halo-(C₁-C₃ alkoxy)carbonyl-C₁-C₃-alkyl with 1 to 13 fluorine, chlorine and/or bromine atoms in each case; (C₁-C₈ alkyl)carbonyl, (C₁-C₈ alkoxy)carbonyl, (C₁-C₄-alkoxy-C₁-C₄-alkyl)carbonyl, (C₃-C₈ cycloalkyl)carbonyl; (C₁-C₆ haloalkyl)carbonyl, (C₁-C₆ haloalkoxy)carbonyl, (halo-C₁-C₄-alkoxy-C₁-C₄-alkyl)carbonyl, (C₃-C₈ halocycloalkyl)carbonyl with 1 to 9 fluorine, chlorine and/or bromine atoms in each case; or -C(=O)C(=O)R⁵, -CONR⁶R⁷ or -CH₂NR⁸R⁹,
whereby R⁵, R⁶, R⁷, R⁸ and R⁹ have the meanings specified in Claim 1,
in the presence of a base and in the presence of a diluent.

4. Media for combating undesired microorganisms, **characterized by** containing at least one haloalkyl carboxamide of the formula (I) according to Claim 1 together with extenders and/or surface-active materials.

5. The use of haloalkyl carboxamides of the formula (I) according to Claim 1 to combat undesired microorganisms.

6. Processes for combating undesired microorganisms, **characterized in that** haloalkyl carboxamides of the formula (I) according to Claim 1 are applied to the microorganisms and/or their environment.

7. Processes for synthesizing materials to combat undesired microorganisms, **characterized in that** haloalkyl carboxamides of the formula (I) according to Claim 1 are mixed with extenders and/or surface-active materials.

## Revendications

1. Haloalkylcarboxamides de formule (I) 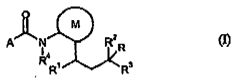 dans lesquels R représente hydrogène ou halogène,
R¹ représente hydrogène ou méthyle,
R² représente méthyle, éthyle ou halogénoalkyle en C₁-C₄ contenant 1 à 9 atomes de fluor, de chlore et/ou de brome,
R³ représente halogène ou halogénoalkyle en C₁-C₄ contenant 1 à 9 atomes de fluor, de chlore et/ou de brome,
R⁴ représente hydrogène, alkyle en C₁-C₈, alkylsulfinyle en C₁-C₆, alkylsulfonyle en C₁-C₆, alcoxy en C₁-C₄-alkyle en C₁-C₄, cycloalkyle en C₃-C₈ ; halogénoalkyle en C₁-C₆, halogénoalkylthio en C₁-C₄, halogénoalkylsulfinyle en C₁-C₄, halogénoalkylsulfonyle en C₁-C₄, halogéno-alcoxy en C₁-C₄-alkyle en C₁-C₄, halogénocycloalkyle en C₃-C₈ contenant à chaque fois 1 à 9 atomes de fluor, de chlore et/ou de brome ; formyle, formyl-alkyle en C₁-C₃, (alkyle en C₁-C₃) carbonyl-alkyle en C₁-C₃, (alcoxy en C₁-C₃)carbonyl-alkyle en C₁-C₃ ; halogéno-(alkyle en C₁-C₃) carbonyl-alkyle en C₁-C₃, halogéno-(alcoxy en C₁-C₃) carbonyl-alkyle en C₁-C₃ contenant à chaque fois 1 à 13 atomes de fluor, de chlore et/ou de brome ; (alkyle en C₁-C₈)carbonyle, (alcoxy en C₁-C₈)carbonyle, (alcoxy en C₁-C₄-alkyle en C₁-C₄)carbonyle, (cycloalkyle en C₃-C₈)carbonyle ; (halogénoalkyle en C₁-C₆) carbonyle, (halogénoalcoxy en C₁-C₆)carbonyle, (halogéno-alcoxy en C₁-C₄-alkyle en C₁-C₄) carbonyle, (halogénocycloalkyle en C₃-C₈)carbonyle contenant à chaque fois 1 à 9 atomes de fluor, de chlore et/ou de brome ; ou -C(=O)C(=O)R⁵, -CONR⁶R⁷ ou - CH₂NR⁸R⁹,
R⁵ représente hydrogène, alkyle en C₁-C₈, alcoxy en C₁-C₈, alcoxy en C₁-C₄-alkyle en C₁-C₄, cycloalkyle en C₃-C₈ ; halogénoalkyle en C₁-C₆, halogénoalcoxy en C₁-C₆, halogéno-alcoxy en C₁-C₄-alkyle en C₁-C₄, halogénocycloalkyle en C₃-C₈ contenant à chaque fois 1 à 9 atomes de fluor, de chlore et/ou de brome,
R⁶ et R⁷ représentent chacun indépendamment l'un de l'autre hydrogène, alkyle en C₁-C₈, alcoxy en C₁-C₄-alkyle en C₁-C₄, cycloalkyle en C₃-C₈ ; halogénoalkyle en C₁-C₈, halogéno-alcoxy en C₁-C₄-alkyle en C₁-C₄, halogénocycloalkyle en C₃-C₈ contenant à chaque fois 1 à 9 atomes de fluor, de chlore et/ou de brome,
R⁶ et R⁷ peuvent également former ensemble avec l'atome d'azote auquel ils sont reliés un hétérocycle saturé de 5 à 8 atomes de cycle, éventuellement substitué une ou plusieurs fois, de manière identique ou différente, par halogène ou alkyle en C₁-C₄, l'hétérocycle pouvant contenir 1 ou 2 hétéroatomes non voisins supplémentaires de la série oxygène, soufre ou NR¹⁰,
R⁸ et R⁹ représentent indépendamment l'un de l'autre hydrogène, alkyle en C₁-C₈, cycloalkyle en C₃-C₈ ; halogénoalkyle en C₁-C₈, halogénocycloalkyle en C₃-C₈ contenant à chaque fois 1 à 9 atomes de fluor, de chlore et/ou de brome,
R⁸ et R⁹ peuvent également former ensemble avec l'atome d'azote auquel ils sont reliés un hétérocycle saturé de 5 à 8 atomes de cycle, éventuellement substitué une ou plusieurs fois, de manière identique ou différente, par halogène ou alkyle en C₁-C₄, l'hétérocycle pouvant contenir 1 ou 2 hétéroatomes non voisins supplémentaires de la série oxygène, soufre ou NR¹⁰,
R¹⁰ représente hydrogène ou alkyle en C₁-C₆,
M représente un phényle substitué à une reprise par R¹¹,
R¹¹ représente hydrogène, fluor, chlore, méthyle, isopropyle, méthylthio ou trifluorométhyle,
A représente le radical de formule (Al) 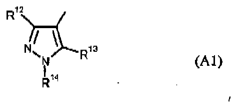 dans laquelle
R¹² représente hydrogène, cyano, halogène, nitro, alkyle en C₁-C₄, alcoxy en C₁-C₄, alkylthio en C₁-C₄, cycloalkyle en C₃-C₆, halogénoalkyle en C₁-C₄, halogénoalcoxy en C₁-C₄ ou halogénoalkylthio en C₁-C₄ contenant à chaque fois 1 à 5 atomes d'halogène, aminocarbonyle ou aminocarbonyl-alkyle en C₁-C₄,
R¹³ représente hydrogène, halogène, cyano, alkyle en C₁-C₄, alcoxy en C₁-C₄ ou alkylthio en C₁-C₄,
R¹⁴ représente hydrogène, alkyle en C₁-C₄, hydroxy-alkyle en C₁-C₄, alcényle en C₂-C₆, cycloalkyle en C₃-C₆, alkylthio en C₁-C₄-alkyle en C₁-C₄, alcoxy en C₁-C₄-alkyle en C₁-C₄, halogénoalkyle en C₁-C₄, halogénoalkylthio en C₁-C₄-alkyle en C₁-C₄, halogénoalcoxy en C₁-C₄-alkyle en C₁-C₄ contenant à chaque fois 1 à 5 atomes d'halogène, ou phényle.

2. Haloalkylcarboxamides de formule (I) selon la revendication 1, dans lesquels
R représente hydrogène, fluor, chlore ou brome,
R¹ représente hydrogène ou méthyle,
R² représente méthyle, éthyle ; ou méthyle, éthyle, n- ou isopropyle, n-, iso-, sec- ou tert-butyle à chaque fois substitué une ou plusieurs fois, de manière identique ou différente, par fluor, chlore ou brome,
R³ représente fluor, chlore, brome, iode ; ou méthyle, éthyle, n- ou isopropyle, n-, iso-, sec- ou tert-butyle à chaque fois substitué une ou plusieurs fois, de manière identique ou différente, par fluor, chlore ou brome,
R⁴ représente hydrogène, alkyle en C₁-C₆, alkylsulfinyle en C₁-C₄, alkylsulfonyle en C₁-C₄, alcoxy en C₁-C₃-alkyle en C₁-C₃, cycloalkyl en C₃-C_{6 ;} halogénoalkyle en C₁-C₄, halogénoalkylthio en C₁-C₄, halogénoalkylsulfinyle en C₁-C₄, halogénoalkylsulfonyle en C₁-C₄, halogéno-alcoxy en C₁-C₃-alkyle en C₁-C₃, halogénocycloalkyle en C₃-C₈ contenant à chaque fois 1 à 9 atomes de fluor, de chlore et/ou de brome ; formyle, formyl-alkyle en C₁-C₃, (alkyle en C₁-C₃)carbonyl-alkyle en C₁-C₃, (alcoxy en C₁-C₃)carbonyl-alkyle en C₁-C₃ ; halogéno-(alkyle en C₁-C₃)carbonyl-alkyle en C₁-C₃, halogéno-(alcoxy en C₁-C₃)carbonyl-alkyle en C₁-C₃ contenant à chaque fois 1 à 13 atomes de fluor, de chlore et/ou de brome ; (alkyle en C₁-C₆)carbonyle, (alcoxy en C₁-C₄)carbonyle, (alcoxy en C₁-C₃-alkyle en C₁-C₃)carbonyle, (cycloalkyle en C₃-C₆)carbonyle ; (halogénoalkyle en C₁-C₄)carbonyle, (halogénoalcoxy en C₁-C₄)carbonyle, (halogéno-alcoxy en C₁-C₃-alkyle en C₁-C₃) carbonyle, (halogénocycloalkyle en C₃-C₆) carbonyle contenant à chaque fois 1 à 9 atomes de fluor, de chlore et/ou de brome ; ou -C(=O)C(=O)R⁵, -CONR⁶R⁷ ou-CH₂NR⁸R⁹,
R⁵ représente hydrogène, alkyle en C₁-C₆, alcoxy en C₁-C₄, alcoxy en C₁-C₃-alkyle en C₁-C₃. cycloalkyle en C₃-C₆; halogénoalkyle en C₁-C₄, halogénoalcoxy en C₁-C₄, halogéno-alcoxy en C₁-C₄-alkyle en C₁-C₃, halogénocycloalkyle en C₃-C₆ contenant à chaque fois 1 à 9 atomes de fluor, de chlore et/ou de brome,
R⁶ et R⁷ représentent chacun indépendamment l'un de l'autre hydrogène, alkyle en C₁-C₆, alcoxy en C₁-C₃-alkyle en C₁-C₃, cycloalkyle en C₃-C₆ ; halogénoalkyle en C₁-C₄, halogéno-alcoxy en C₁-C₃-alkyle en C₁-C₃, halogénocycloalkyle en C₃-C₆ contenant à chaque fois 1 à 9 atomes de fluor, de chlore et/ou de brome,
R⁶ et R⁷ peuvent également former ensemble avec l'atome d'azote auquel ils sont reliés un hétérocycle saturé de 5 ou 6 atomes de cycle, éventuellement substitué une à quatre fois, de manière identique ou différente, par halogène ou alkyle en C₁-C₄, l'hétérocycle pouvant contenir 1 ou 2 hétéroatomes non voisins supplémentaires de la série oxygène, soufre ou NR¹⁰,
R⁸ et R⁹ représentent indépendamment l'un de l'autre hydrogène, alkyle en C₁-C₆, cycloalkyle en C₁-C₆ ; halogénoalkyle en C₁-C₄, halogénocycloalkyle en C₃-C₆ contenant à chaque fois 1 à 9 atomes de fluor, de chlore et/ou de brome,
R⁸ et R⁹ peuvent également former ensemble avec l'atome d'azote auquel ils sont reliés un hétérocycle saturé de 5 ou 6 atomes de cycle, éventuellement substitué une ou plusieurs fois, de manière identique ou différente, par halogène ou alkyle en C₁-C₄, l'hétérocycle pouvant contenir 1 ou 2 hétéroatomes non voisins supplémentaires de la série oxygène, soufre ou NR¹⁰,
R¹⁰ représente hydrogène ou alkyle en C₁-C₄,
M représente un des cycles suivants 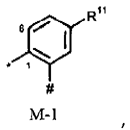 la liaison marquée avec «*» étant reliée avec l'amide, la liaison marquée avec «#» étant reliée avec le radical haloalkyle,
R¹¹ représente hydrogène, fluor, chlore, méthyle ou trifluorométhyle,
A représente le radical de formule (Al) 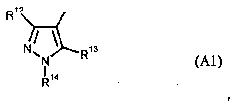 dans laquelle
R¹² représente hydrogène, cyano, fluor, chlore, brome, iode, méthyle, éthyle, isopropyle, méthoxy, éthoxy, méthylthio, éthylthio, cyclopropyle, halogénoalkyle en C₁-C₂, halogénoalcoxy en C₁-C₂ contenant à chaque fois 1 à 5 atomes de fluor, de chlore et/ou de brome, trifluorométhylthio, difluorométhylthio, aminocarbonyle, aminocarbonylméthyle ou aminocarbonyléthyle,
R¹³ représente hydrogène, fluor, chlore, brome, iode, méthyle, éthyle, méthoxy, éthoxy, méthylthio ou éthylthio,
R¹⁴ représente hydrogène, méthyle, éthyle, n-propyle, isopropyle, halogénoalkyle en C₁-C₂ contenant 1 à 5 atomes de fluor, de chlore et/ou de brome, hydroxyméthyle, hydroxyéthyle, cyclopropyle, cyclopentyle, cyclohexyle ou phényle.

3. Procédé de fabrication des haloalkylcarboxamides de formule (I) selon la revendication 1, **caractérisé en ce que**
a) des dérivés d'acides carboxyliques de formule (II) 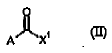 dans laquelle
A a les significations indiquées dans la revendication 1 et
X¹ représente halogène ou hydroxy,
sont mis en réaction avec des dérivés d'aniline de formule (III) 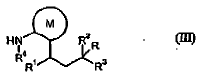 dans laquelle
R, R¹, R², R³, R⁴ et M ont les significations indiquées dans la revendication 1,
éventuellement en présence d'un catalyseur, éventuellement en présence d'un agent de condensation,
éventuellement en présence d'un liant d'acide et éventuellement en présence d'un diluant,
ou
b) des hexylcarboxanilides de formule (I-a) 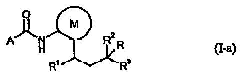 dans laquelle
R, R¹, R², R³, M et A ont les significations indiquées dans la revendication 1,
sont mis en réaction avec des halogénures de formule (IV) 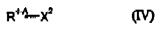 dans laquelle
X² représente chlore, brome ou iode,
R^{4-A} représente alkyle en C₁-C₈ alkylsulfinyle en C₁-C₆, alkylsulfonyle en C₁-C₆, alcoxy en C₁-C₄-alkyle en C₁-C₄, cycloalkyle en C₃-C₈ ; halogénoalkyle en C₁-C₆, halogénoalkylthio en C₁-C₄, halogénoalkylsulfinyle en C₁-C₄, halogénoalkylsulfonyle en C₁-C₄, halogéno-alcoxy en C₁-C₄-alkyle en C₁-C₄, halogénocycloalkyle en C₃-C₈ contenant à chaque fois 1 à 9 atomes de fluor, de chlore et/ou de brome ; formyle, formyl-alkyle en C₁-C₃, (alkyle en C₁-C₃) carbonyl-alkyle en C₁-C₃, (alcoxy en C₁-C₃) carbonyl-alkyle en C₁-C₃ ; halogéno-(alkyle en C₁-C₃)carbonyl-alkyle en C₁-C₃, halogéno-(alcoxy en C₁-C₃)carbonyl-alkyle en C₁-C₃ contenant à chaque fois 1 à 13 atomes de fluor, de chlore et/ou de brome ; (alkyle C₁-C₈)carbonyle, (alcoxy en C₁-C₆) carbonyle, (alcoxy en C₁-C₄-alkyle en C₁-C₄)carbonyle, (cycloalkyle en C₃-C₈)carbonyle ;(halogénoalkyle en C₁-C₆)carbonyle, (halogénoalcoxy en C₁-C₆) carbonyle, (halogéno-alcoxy en C₁-C₄-alkyle en C₁-C₄)carbonyle, (halogénocycloalkyle en C₁-C₈)carbonyle contenant à chaque fois 1 à 9 atomes de fluor, de chlore et/ou de brome ; ou -C(=O)C(=O)R⁵,-CONR⁶R⁷ ou -CH₂NR⁸R⁹,
R⁵, R⁶, R⁷, R⁸ et R⁹ ayant les significations indiquées dans la revendication 1,
en présence d'une base et en présence d'un diluant.

4. Agent de lutte contre les microorganismes indésirables, **caractérisé par** une teneur en au moins un haloalkylcarboxamide de formule (I) selon la revendication 1 en plus d'extendeurs et/ou de substances tensioactives.

5. Utilisation d'haloalkylcarboxamides de formule (I) selon la revendication 1 pour lutter contre les microorganismes indésirables.

6. Procédé de lutte contre les microorganismes indésirables, **caractérisé en ce que** des haloalkylcarboxamides de formule (I) selon la revendication 1 sont appliqués sur les microorganismes et/ou leur habitat.

7. Procédé de fabrication d'agents pour lutter contre les microorganismes indésirables, **caractérisé en ce que** des haloalkylcarboxamides de formule (I) selon la revendication 1 sont mélangés avec des extendeurs et/ou des substances tensioactives.
